# EUROPEAN PATENT APPLICATION

(11) **EP 2 915 810 A1**
(43) Date of publication of application: **09.09.2015**
(21) Application number: 13851443.5
(22) Date of filing: 31.10.2013
(51) Int. Cl.: C07D 401/14, C07D 401/04, A61K 31/4709, A61P 35/00, A61P 35/02, A61P 37/06

(54) **N-(5-(QUINOLIN-6-YL)PYRIDIN-3-YL) BENZSULFAMIDE DERIVATIVES, PREPARATION METHOD AND THERAPEUTIC USE THEREOF**

(30) Priority: 01.11.2012 CN 201210430580
(71) Applicant: The Second Military Medical University, PLA, Shanghai 200433 (CN)
(72) Inventor: ZHU, Ju, Shanghai 200433 (CN); SONG, Yunlong, Shanghai 200433 (CN); HAN, Jinsong, Shanghai 200433 (CN); CHEN, Ying, Shanghai 200433 (CN); LV, Jiaguo, Shanghai 200433 (CN); ZHOU, Youjun, Shanghai 200433 (CN)
(74) Representative: Von Kreisler Selting Werner - Partnerschaft von Patentanwälten und Rechtsanwälten mbB
(86) International application number: PCT/CN2013/086349
(87) International publication number: WO 2014/067473

(57) **Abstract**

The present invention relates to the field of medical technology, and in particular, to a variety of substituted N-(5-(quinolin -6-yl)pyridin -3 -yl) bezsulfamide derivatives represented by formula (1) (groups therein are as defined in the specification).The compounds according to the present invention have favorable anti-tumor activity against human lung cancer, colon cancer, liver cancer, breast cancer and glioblastoma multiforme, which contribute to development of highly effective, low toxic and high specific anti-tumor drugs, and have high value of development. The present invention also relates to a composition, preparation method and use thereof in the preparation of anti-tumor drugs.

## Description

### FIELD OF THE INVENTION

The present invention relates to the field of medical technology, and in particular, to a variety of substituted N-(5-(quinolin-6-yl) pyridin-3-yl) benzsulfamide derivatives, preparation method, and composition thereof. The present invention further relates to use of such compounds in the preparation of a medicine for the treatment of one or more of the following diseases: autoimmune diseases, inflammatory diseases, cardiovascular diseases, neurodegenerative diseases, allergic reaction, asthma, pancreatitis, multiple organ failure, kidney diseases, platelet aggregation, cancer, sperm motility, graft rejection, graft rejection and lung damage, especially cancer. The compounds have outstanding broad-spectrum inhibition activity against tumor cells such as human lung cancer, colon cancer, liver cancer, breast cancer, leukemia, melanoma, ovarian cancer, renal cancer, prostate cancer, cancer of the central nervous system, etc.

### BACKGROUND OF INVENTION

Phosphatidylinositol 3-kinase (PI3K) is a member of lipid kinase family, which can regulate cell metabolism and growth through 3 -phosphorylation of phosphatidylinositol to produce lipid phosphatidylinositol triphosphate (PIP3).As an important second messenger, PIP3 can combine with downstream effectors (particularly Akt), thus resulting in membrane recruitment and phosphorylation. Abnormal activation of PI3K can cause a range of reactions, including cell growth, proliferation or metastasis, transition from epithelial cells to mesenchymal cells and angiogenesis.

Eukaryotes response to stimulates such as growth factor receptors, hormones, cytokines, etc, activating different signal transduction pathways of intracellular, in which PI3K / mTOR is an important signal transduction pathway involved in cell proliferation and apoptosis. Studies have shown that this pathway controls biological processes of common tumors such as breast cancer, colorectal cancer, prostate cancer, ovarian cancer, liver cancer, and lymphoma, including apoptosis, transcription, translation, metabolism, angiogenesis, and cell cycle regulation, and provides a new target. Thereby inhibiting the signal pathway has become a hot spot for cancer prevention and targeted cancer therapy, for each of the kinase of the signaling pathway, there are a variety of inhibitors in pre-clinical and clinical studies.

PI3K can be divided into type I, II and III kinase, and type-I PI3K can be once again divided into two groups according to their structure and the receptor making it activated, i.e., I_{A} and I_{B}, and the former can be divided into PI3Kα, PI3Kß and PI3Kδ and all of them can be activated by receptor tyrosine kinase, while the latter has only one member PI3Kγ which could be activated by G protein-coupled receptor. Recent studies have shown that the inhibition effect of PI3Kα has an indispensable influence on growth inhibition of malignant cell lines. In addition, the non-PI3Kα type I-PI3K isomers (PI3K ß and δ) is capable of inducing oncogenic transformation of cells, suggesting that non-specific isomers inhibitors may enhance the therapeutic effect of specific inhibitors. It is also found that selective inhibitor of PI3Kδ may enhance the impact of radiation effect on tumor growth.

PI3K inhibitor has important clinical value as it can avoid feedback activation of Akt generated by the inhibition of mTOR. Presently PI3K inhibitor acts mainly to the catalytic subunit p110, and it can be divided into three categories according to the specificity of its role, which are non-specific inhibitors targeting the catalytic subunit p110 of PI3K, p110 subtype-specific inhibitors, and other type inhibitors.

Wortmannin and LY294002 are PI3K inhibitors of first generation, which are both of little or no selectivity to isozyme, thus with greater toxicity. Many compounds targeting PI3K are in clinical trials, most of which are dual inhibitors of PI3K/mTOR, showing good therapeutic potential.

In recent years, cancer has become the first cause of death in urban and rural residents in China, with the mortality rate showing a rising trend; nowadays chemotherapy is the primary method of treatment of tumors. The conventional cytotoxic anti-tumor drugs have drawbacks such as of low selectivity, great side effects and other serious drawbacks, while PI3K inhibitor targets the tumor cells, so it is expected to reduce the above disadvantages.

### DESCRIPTION OF THE INVENTION

The present invention first relates to a variety of substituted N-(5-(quinolin-6-yl) pyridin-3-yl) benzsulfamide derivatives represented by formula (1), or the solvate, pharmaceutically acceptable salt, pro-drug or polymorph thereof: 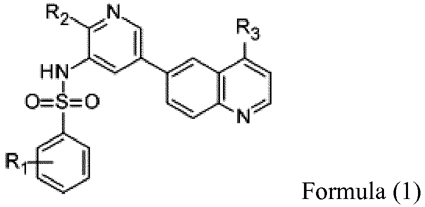 wherein,
R₁ group may be independently selected from the group consisting of: hydrogen, halogen, nitrile group, nitro group, hydroxyl group, carboxyl group, substituted or unsubstituted C1-8 acyl group, substituted or unsubstituted amino group, substituted or unsubstituted C1-8 alkyl group, substituted or unsubstituted C1-8 alkoxy group, which may be single, double or multi-substituted;
R₂ group may be independently selected from the group consisting of: hydrogen, substituted or unsubstituted C1-8 alkoxy group, or halogen;
R₃ group is selected from any of the following groups: 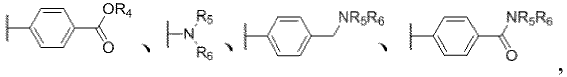 wherein, R₄, R₅, R₆ may be independently selected from the group consisting of: hydrogen, substituted or unsubstituted C1-8 straight or branched alkyl group, R₅ and R₆ may form a ring, with a proviso that R₅ and R₆ are not simultaneously hydrogen; R₄ and R₅ may also be-(CH₂)ₙNR₇R₈, wherein n is 1-8, R₇ and R₈ may be independently selected from the group consisting of: hydrogen, substituted or unsubstituted C1-8 straight or branched alkyl group, R₇ and R₈ may also form a ring;
when R₅ and R₆ form a ring, or R₇ and R₈ form a ring, they may form a nitrogen-containing saturated heterocyclic ring or an aromatic heterocyclic ring with the nitrogen attached, while the heterocyclic ring may be substituted;

The term "substituted"means a group substitution by one or more of the following substituents: C1-5 alkyl, C2-5 alkenyl, C2-5 alkynyl, C1-5 alkoxy, halogen, nitro, cyano, hydroxyl, amino, carboxyl, or oxo.

Preferably, the compound of formula (I), wherein R₁ group is mono-, di- or poly-substituted by halogen. More preferably, R₁ group is 2, 4-difluoro, 4-fluoro or 2-fluoro-substituted.

Preferably, the compound of formula (I), wherein R₂ group is methoxy or halogen.

Preferably, the compound of formula (I), when R₅ and R₆ form a ring, or R7 and R8 form a ring, thus forming nitrogen-containing saturated heterocyclic ring or aromatic heterocyclic ring with the attached nitrogen, wherein the saturated heterocyclic ring or aromatic heterocyclic ring comprises: pyrrolidine, piperidine, piperazine, morpholine, imidazole, 2-methylimidazole, pyrazole, 1*H*-1*,* 2, 4- triazole.

The present invention also provides a method for preparing a compound of formula (IV), comprising the following steps of: 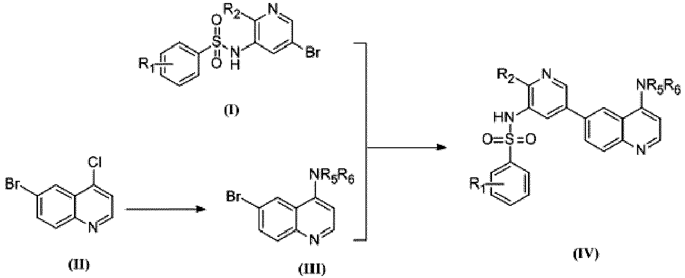
(1) Preparation of intermediate (III)
   dissolving 6-bromo-4-chloro-quinoline **(II)** in DMF, adding various substituted amines, heating and reacting to get intermediate **(III);**
(2) Preparation of desired product **(IV)**
   intermediate **(III)** is dissolved in dioxane, and bis (pinacolato) diboron , potassium acetate and palladium catalyst are added;
   heating and refluxing under inert gas protected condition until raw materials point of TLC testing disappeares; then the reaction is stopped; adding various substituted brominated aromatic compounds **(I)** and the other part of palladium catalyst and K₂CO₃ solution, heating and refluxing under inert gas protected condition, thereby forming the desired product **(IV).**

The present invention also provides a method for preparing a compound of formula **(VIII),** comprising the following steps of: 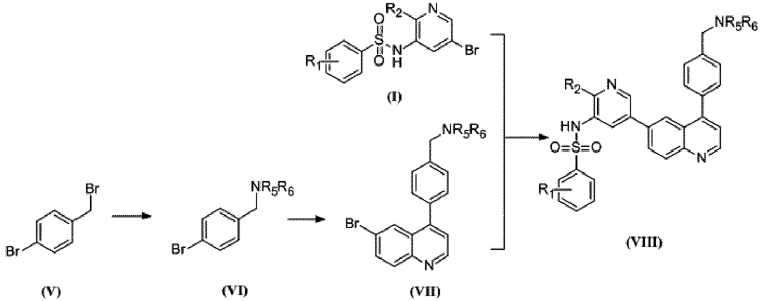
(1) Preparation of compound **(VI)**
   dissolving bromine bromide **(V)** in DMF, adding various nitrogen substituted compounds, stirring overnight under nitrogen protected condition at room temperature to generate compound **(VI);**
(2) Preparation of intermediate **(VII)**
   nitrogen-substituted benzyl derivatives **(VI)** are dissolved in dioxane; and bis(pinacolato) diboron, potassium acetate and a palladium catalyst are added; heating and refluxing for several hours under argon protected condition until raw materials point of TLC testing disappears, then stop the reaction; 6-bromo-4-iodo-quinoline, the other part of palladium catalyst and K₂CO₃ solution are added into the mixed system, the reaction is stopped after heating and refluxing for several hours under argon protected condition, then stop the reaction, thereby forming the desired product **(VII);**
(3) Preparation of desired product **(VIII)**
   intermediate **(VII)** is dissolved in dioxane, and bis (pinacolato) diboron, potassium acetate and a palladium catalyst are added; heating and refluxing for several hours under argon protected condition until raw materials point of TLC testing disappears, then the reaction is stopped; adding brominate substituted aromatic compound (I), palladium catalyst and K₂CO₃ solution into the mixed system; heating and refluxing for several hours under argon protected condition, thereby forming the desired product **(VIII).**

The present invention also provides a method for preparing a compound of formula **(XII),** comprising the following steps of: 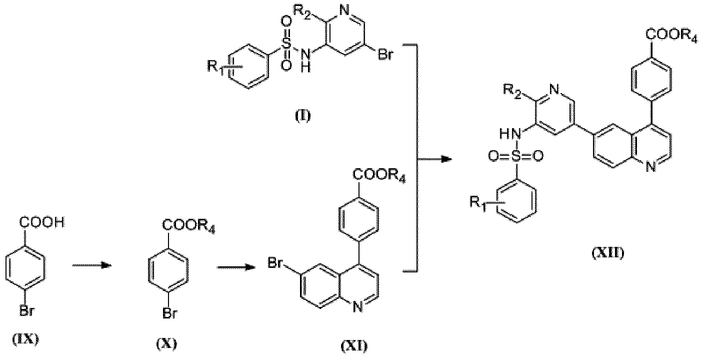
(1) Preparation of p-bromobenzoate ester derivative **(X)**
   para-bromobenzoic acid **(IX)** is dissolved in alcohols, and a few droplets of concentrated sulfuric acid is added dropwise, followed by stirring under heating to reflux to form compound **(X);**
(2) Preparation of intermediate **(XI)**
   para-bromobenzoic acid ester derivatives **(X)** are dissolved in dioxane, and bis(pinacolato) diboron, potassium acetate and a palladium catalyst are added; heating and refluxing for several hours under argon protected condition until raw material point of TLC testing disappears, then the reaction is stopped; adding 6-bromo-4-iodo-quinoline, the other part of palladium catalyst and K₂CO₃ solution into the mixed system, heating and refluxing several hours under argon protected condition, and stopping the reaction to obtain the intermediate **(XI);**
(3) Preparation of desired product **(XII)**
   intermediate **(XI)** is dissolved in dioxane, and bis (pinacolato) diboron, potassium acetate and a palladium catalyst are added; heating and refluxing for several hours under argon protected condition until raw material point of TLC testing disappears, then the reaction is stopped; adding substituted brominated aromatic compounds **(I),** palladium catalyst and K₂CO₃ solution into the mixed system, heating and refluxing for several hours under argon protected condition, and stopping the reaction to obtain the intermediate **(XII).**

The present invention also provides a method for preparing a compound of formula **(XVI),** comprising the following steps of : 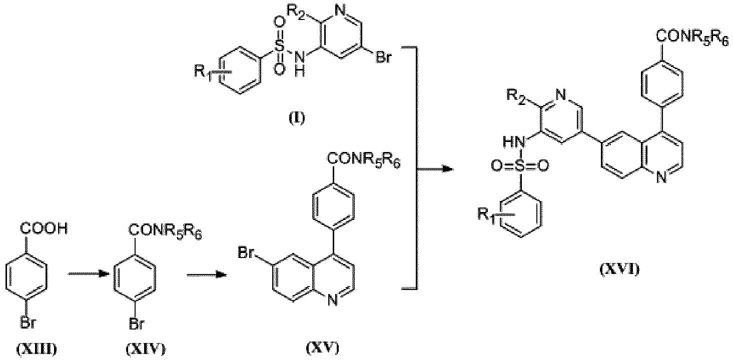
(1) Preparation of para-bromobenzamide derivative **(XIV)**
   para-bromobenzoic acid **(XIII)** is dissolved in dichloromethane, EDC/HCl and DMAP are then added; after reacting for two hours, adding another raw material various amine, heating overnight at the room temperature to form compound **(XIV);**
(2) Preparation of the intermediate **(XV)**
   para-bromobenzamide derivatives **(XIV)** are dissolved in dioxane, and bis (pinacolato) diboron, potassium acetate and palladium catalyst are added; heating and refluxing for several hours under argon protected condition until raw materials point of TLC testing disappears, then the reaction is stopped; adding 6-bromo-4-iodo-quinoline and another part of palladium catalyst and K₂CO₃ solution into the mixed system, heating and refluxing for several hours under argon protected condition, and stopping the reaction to obtain the intermediate **(XV);**
(3) Preparation of desired product **(XVI)**
   intermediate **(XV)** is dissolved in dioxane, and bis (pinacolato) diboron, potassium acetate and a palladium catalyst are added; heating and refluxing for several hours under argon protected condition until raw material point of TLC testing disappears, then the reaction is stopped; adding brominated aromatic compound **(I),** palladium catalyst and K₂CO₃ solution into the mixed system, heating and refluxing for several hours under argon protected condition, and stopping the reaction to obtain the intermediate **(XII).**

More particularly, compounds relative to the present invention are as follows:
(1)N-(5-(4-(1*H*-1, 2, 4-triazole-1-yl)quinolin-6-yl)-2-methoxypyridine-3-yl)-2, 4-difluorobenzenesulfonamide;
(2)N-(2-methoxy-5-(4-(pyridin-1-yl)quinolin-6-yl)pyridin-3-yl)-2, 4-difluorobenzenesulfonamide;
(3)N-(2-methoxy-5-(4-morpholine quinolin-6-yl)pyridin-3-yl)- 2, 4-difluorobenzenesulfonamide;
(4) N-(5-(4-chlorinequinolin-6-yl)-2-methoxypyridine-3-yl)-2, 4-difluorobenzenesulfonamide;
(5)N-(5-(4-(4-((diethylamine)methyl)phenyl)quinolin-6-yl)-2-methoxypyridine-3-yl)-2, 4-difluorobenzenesulfonamide;
(6)N-(2-methoxy-5-(4-(p-methylphenyl)quinolin-6-yl)pyridin-3-yl)-2, 4-difluorobenzenesulfonamide;
(7) 4-(6-(5-(2, 4-difluorobenzenesulfonamideyl)-6-methoxypyridine-3-yl)quinolin-4-yl)methyl benzoate;
(8) 4-(6-(5-(2, 4-difluorobenzenesulfonamideyl)-6-methoxypyridine-3-yl)quinolin-4-yl) ethyl benzoate;
(9)N-(5-(4-(4-((dimethylamine)methyl)phenyl)quinolin-6-yl)-2-methoxypyridine-3-yl)-2, 4-difluorobenzenesulfonamide;
(10)N-(5-(4-(4-((1*H*-1,2, 4-triazole-1-yl) methyl)phenyl) quinolin-6-yl)-2 -methoxypyridine-3-yl)-2, 4-difluorobenzenesulfonamide;
(11) N-(2-methoxy-5-(4-(4-(pyrrolidinyl-1-carbonyl)phenyl) quinolin-6-yl)pyridin-3-yl)- 2, 4-difluorobenzenesulfonamide;
(12) N-(5-(4-(4-((tetrahydropyridinyl-1-yl)methyl)phenyl)quinolin-6-yl)-2- methoxypyridine-3-yl)-2, 4-difluorobenzenesulfonamide;
(13)N-(5-(4-(4-((morpholino-1-yl)methyl)phenyl)quinolin-6-yl)-2-methoxypyridine-3-yl)-2, 4-difluorobenzenesulfonamide;
(14)N-(5-(4-(4-((piperidine-1-yl)methyl)phenyl)quinolin-6-yl)-2-methoxypyridine-3-yl)-2, 4-difluorobenzenesulfonamide;
(15) N-(5-(4-(4-((1*H*-imidazole-1-yl)methyl)phenyl)quinolin-6-yl)-2-methoxypyridine-3-yl)-2, 4-difluorobenzenesulfonamide;
(16) N-(5-(4-(4-((2-methyl-1*H*-imidazole-1-yl)methyl)phenyl)quinolin-6-yl) -2-methoxypyridine-3-yl)-2, 4-difluorobenzenesulfonamide;
(17) N-(5-(4-(4-(((2-hydroxyethyl)(methyl)amino)methyl)phenyl)quinolin-6-yl) -2-methoxypyridine-3-yl)-2, 4-difluorobenzenesulfonamide;
(18) N-(5-(4-(4-((4-isopropylpiperazine-1-yl)methyl)phenyl)quinolin -6-yl)-2-methoxypyridine-3-yl)-2, 4-difluorobenzenesulfonamide;
(19) N-(5-(4-(4-((4-(2-hydroxyethyl)piperazine-1-yl)methyl)phenyl)quinolin- 6-yl)-2-methoxypyridine-3-yl)-2, 4-difluorobenzenesulfonamide;
(20) N-(5-(4-(4-((di(2-hydroxyethyl)amino)methyl)phenyl)quinolin-6-yl)-2-methoxypyridine-3-yl)-2, 4-difluorobenzenesulfonamide;
(21) N-(5-(4-(4-((1*H*-pyrazole-1-yl)methyl)phenyl)quinolin-6-yl)-2-methoxypyridine-3-yl)-2, 4-difluorobenzenesulfonamide;
(22) N-(5-(4-(4-((cyclohexyl(ethyl)amino)methyl)phenyl)quinolin-6-yl)-2-methoxypyridine-3-yl)-2, 4-difluorobenzenesulfonamide;
(23) N-(5-(4-(4-((4-methylpiperazine-1-yl)methyl)phenyl)quinolin-6-yl)-2-methoxypyridine-3-yl)-2, 4-difluorobenzenesulfonamide;
(24) N-(5-(4-(4-(((2-(dimethylamino)ethyl)(methyl)amino))methyl)phenyl)quinolin-6-yl) -2-methoxypyridine-3-yl)-2, 4-difluorobenzenesulfonamide;
(25) N-(5-(4-(1*H* -1, 2, 4-triazole-1-yl)quinolin-6-yl)-2- methoxypyridine-3-yl)- 4-fluorobenzenesulfonamide;
(26) N-(5-(4-(1*H* -1, 2, 4- triazole-1-yl) quinolin-6-yl)- 2-chlorinepyridin- 3-yl)- 2, 4-difluorobenzenesulfonamide;
(27) N-(5-(4-(1*H* -1, 2, 4-triazole-1-yl)quinolin-6-yl)-2- chlorinepyridin-3-yl) -4-fluorobenzenesulfonamide;
(28) N-(5-(4-(4-((morpholino-1-yl)methyl)phenyl)quinolin-6-yl)-2-chlorinepyridin-3-yl)- 4-fluorobenzenesulfonamide;
(29) N-(5-(4-(4-((morpholino-1-yl)methyl)phenyl)quinolin-6-yl)-2-methoxypyridine- 3-yl)-4-fluorobenzenesulfonamide;
(30) N-(5-(4-(4-((morpholino-1-yl)methyl)phenyl)quinolin-6-yl)-2-chlorinepyridin-3-yl)- 2, 4-difluorobenzenesulfonamide;
(31) N-(5-(4-(4-((dimethylamine)methyl)phenyl)quinolin-6-yl)-2-methoxypyridine-3-yl)- 4-fluorobenzenesulfonamide;
(32) N-(5-(4-(4-((dimethylamine)methyl)phenyl)quinolin-6-yl)-2-chlorinepyridin-3-yl)-2, 4-difluorobenzenesulfonamide;
(33) N-(5-(4-(4-((1*H-*1, 2, 4-triazole-1-yl)methyl)phenyl)quinolin-6-yl)-2-chlorinepyridin-3-yl) -4-fluorobenzenesulfonamide;
(34) N-(5-(4-(4-((1*H*-1, 2, 4-triazole-1-yl)methyl)phenyl)quinolin-6-yl) -2-methoxypyridine -3-yl)-4-fluorobenzenesulfonamide;
(35) N-(5-(4-(4-((1*H*-1, 2, 4-triazole-1-yl)methyl)phenyl)quinolin-6-yl)-2-chlorinepyridin-3-yl) -2, 4-difluorobenzenesulfonamide;
(36) N-(5-(4-(4-(((2-hydroxyethyl)(methyl)amino)methyl)phenyl)quinolin-6-yl)-2-chlorinepyridin-3-yl)-4-fluorobenzenesulfonamide;
(37) N-(5-(4-(4-(((2-hydroxyethyl)(methyl)amino)methyl)phenyl)quinolin-6-yl) -2-chlorinepyridin-3-yl)-2, 4-difluorobenzenesulfonamide; and
(38) N-(5-(4-(4-(((2-hydroxyethyl)(methyl)amino)methyl)phenyl)quinolin-6-yl) -2-methoxypyridine-3-yl)-4-fluorobenzenesulfonamide.

The chemical structures of part of the compounds of the present invention and the ¹H-NMR data are shown in Table 1, MS, melting point, and infrared data in Table 2.

**Table 1 the chemical structures of part of the compounds the ¹H-NMR data**

| Compound number | structure | ¹H-NMR data |
|---|---|---|
| **1** | 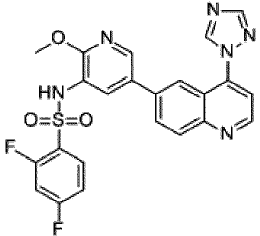 | ¹H NMR (300 MHz, DMSO) δ 10.48 (s, 1H), 9.36 (s, 1H), 9.10 (d, J = 4.7 Hz, 1H), 8.51 (s, 1H), 8.44 (d, J = 2.3 Hz, 1H), 8.31 (d, J = 1.9 Hz, 1H), 8.27 (s, 1H), 8.20 (dd, J = 8.9, 1.9 Hz, 1H), 7.96 (d, J = 2.3 Hz, 1H), 7.86 (d, J = 4.7 Hz, 1H), 7.88-7.69 (m, 1H), 7.62-7.53 (m, 1H), 7.21 (td, J = 8.4, 2.2 Hz, 1H), 3.67 (s, 3H). |
| **2** | 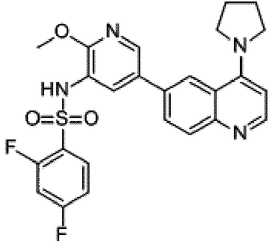 | ¹H NMR (300 MHz, CDCl₃) δ 8.60 (s, 1H), 8.49 (d, J = 2.4 Hz, 1H), 8.46 (d, J = 7.2 Hz, 1H), 8.21 (dd, J = 8.9, 1.3 Hz, 1H), 8.04 (d, J = 2.4 Hz, 1H), 7.97 (d, J = 8.7 Hz, 1H), 7.79-7.69 (m, 1H), 7.63-7.54 (m, 1H), 7.23 (td, J = 8.8, 2.3 Hz, 1H), 6.81 (d, J = 7.2 Hz, 1H), 4.04 (br s, 4H), 3.67 (s, 3H), 2.07 (br s, 4H). |
| **3** | 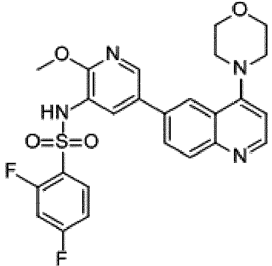 | ¹H NMR (600 MHz, DMSO) δ 10.39 (s, 1H), 8.72 (d, J = 5.0 Hz, 1H), 8.44 (s, 1H), 8.14 (d, J = 2.0 Hz, 1H), 8.05 (d, J = 8.7 Hz, 1H), 7.98 (td, J = 9.1, 2.3 Hz, 2H), 7.78 (m, 1H), 7.57 (m, 1H), 7.22 (td, J = 8.7, 2.3Hz, 1H), 7.05 (d, J = 5.0 Hz, 1H), 3.91 (t, J = 4.4 Hz, 4H), 3.71 (s, 3H), 3.23 (t, J = 4.4 Hz, 4H). |
| **4** | 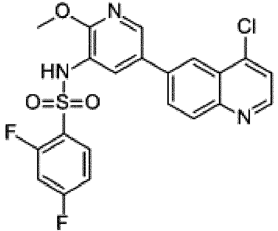 | ¹H NMR (300 MHz, CDCl₃) δ 8.82 (d, J = 4.8 Hz, 1H), 8.28 (d, J = 2.0 Hz, 1H), 8.24 (d, J = 2.2 Hz, 1H), 8.09 (d, J = 2.2 Hz, 1H), 7.93 (m, 2H), 7.58 (d, J = 4.8 Hz, 1H), 7.32 (s, 1 ), 6.98 (m, 2H), 4.00 (s, 3H) |
| **5** | 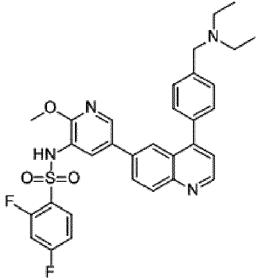 | ¹H NMR (300 MHz, DMSO) δ 8.96 (d, J = 4.5 Hz, 1H), 8.28 - 8.16 (m, 2H), 8.09 - 7.99 (m, 2H), 7.81 (d, J = 2.1 Hz, 1H), 7.76 - 7.55 (m, 6H), 7.52 (d, J = 4.1 Hz, 1H), 7.12 (t, J = 8.7 Hz, 1H), 3.84 (s, 2H), 3.68 (s, 3H), 2.67 (q, J = 7.1 Hz, 4H), 1.07 (t, J = 7.1 Hz, 6H) |
| **6** | 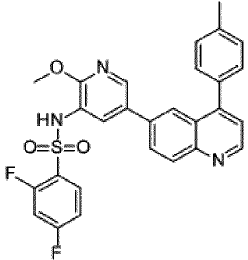 | ¹H NMR (300 MHz, DMSO) δ 10.35 (s, 1H), 8.94 (d, J = 4.4 Hz, 1H), 8.31 (m, 1H), 8.19 (d, J = 8.7 Hz, 1H), 8.03 (m, 2H), 7.83 (m, 1H), 7.70 (dd, J = 15.5, 8.3 Hz, 1H), 7.58-7.40 (m, 6H), 7.14 (m , 1H), 3.65 (s, 3H), 2.43 (s, 3H) |
| **7** | 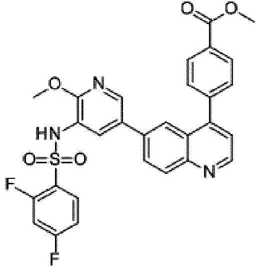 | ¹H NMR (300 MHz, CDCl₃) δ 9.00 (d, J = 4.3 Hz, 1H), 8.32 (d, J = 9.5 Hz, 1H), 8.26 (d, J = 8.4 Hz, 2H), 8.07 (d, J = 2.2 Hz, 1H), 7.97 (d, J = 2.2 Hz, 1H), 7.89 (dd, J = 6.3, 1.9 Hz, 2H), 7.78 (td, J = 8.4, 5.8 Hz, 1H), 7.65 (d, J = 8.4 Hz, 2H), 7.43 (d, J = 4.3 Hz, 1H), 6.97 - 6.81 (m, 2H), 4.00 (s, 3H), 3.95 (s, 3H). |
| **8** | 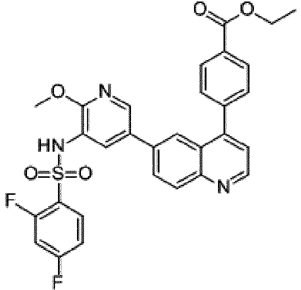 | ¹H NMR (300 MHz, CDCl₃) δ 9.00 (d, J = 4.3 Hz, 1H), 8.33 (d, J = 9.0 Hz, 1H), 8.27 (d, J = 8.2 Hz, 2H), 8.07 (d, J = 2.2 z, 1H), 7.97 (d, J = 2.2 Hz, 1H), 7.90 (dd, J = 6.3, 1.9 Hz, 2H), 7.78 (td, J = 8.4, 5.8 Hz, 1H), 7.64 (d, J = 8.2 Hz, 2H), 7.44 (d, J = 4.3 Hz, 1H), 6.91 (m, 2H), 4.46 (q, J = 7.1 Hz, 2H), 3.95 (s, 3H), 1.45 (t, J = 6.7 Hz, 3H). |
| **9** | 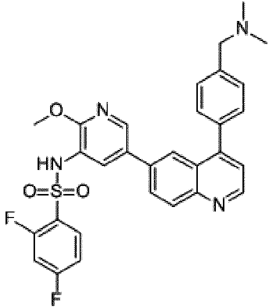 | ¹H NMR (300 MHz, CDCl₃) δ 8.97 (d, *J* = *4.5* Hz, 1H), 8.26 (d, *J* = 8.7 Hz, 1H), 8.11 (d, *J* = 2.2 Hz, 1H), 8.00 (dd, *J* = 9.1, 2.1 Hz, 2H), 7.82 (m, 2H), 7.57 (q, *J* = 8.2 Hz, 4H), 7.39 (d, *J* = 4.5 Hz, 1H), 6.91 (m, 2H), 3.95 (s, 3H), 3.67 (s, 2H), 2.42 (s, 6H). |
| **10** | 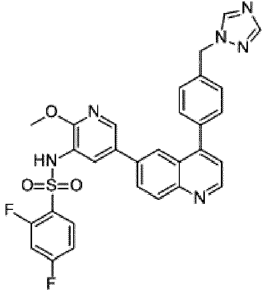 | ¹H NMR (300 MHz, CDCl₃) δ 8.97 (d, *J*=4.2 Hz, 1H), 8.29 (d, *J* = 9.1 Hz, 1H), 8.08 (m, 2H), 7.97 (d, *J* = 9.8 Hz, 2H), 7.87 (d, *J =* 7.4 Hz, 1H), 7.76 (dd, *J* = 13.8, 7.7 Hz, 1H), 7.58 (d, *J* = 6.7 Hz, 2H), 7.48 (d, *J* = 6.4 Hz, 2H), 7.37 (d, *J* = 11.5 Hz, 2H), 6.89 (dt, *J* = 15.2, 8.3 Hz, 2H), 5.51 (s, 2H), 3.94 (s, 3H). |
| **11** | 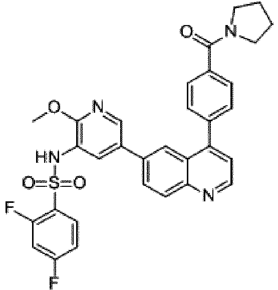 | ¹H NMR (300 MHz, CDCl₃) δ 8.99 (d, *J* = 3.9 Hz, 1H), 8.37 (d, *J* = 8.3 Hz, 1H), 8.11 (d, *J* = 2.3 Hz, 1H), 7.99 (d, *J* = 2.0 Hz, 2H), 7.92 (d, *J* = 8.9 Hz, 1H), 7.77 (m, 3H), 7.62 (d, *J* = 8.0 Hz, 2H), 7.45 (d, *J* = 4.3 Hz, 1H), 6.94 (t*, J* = 8.2 Hz, 2H), 3.96 (s, 3H), 3.65 (dt, *J* = 12.1, 6.4 Hz, 4H), 1.98 (m, 4H). |
| **12** | 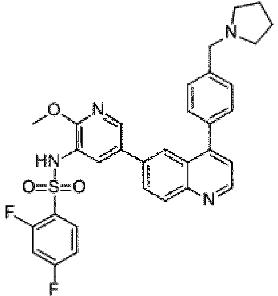 | ¹H NMR (600 MHz, CDCl₃) δ 8.95 (d, *J* = 4.4 Hz, 1H), 8.24 (d, *J* = 8.7 Hz, 1H), 8.10 (d, *J* = 2.2 Hz, 1H), 8.02 (d, *J* = 1.8 Hz, 1H), 7.98 (d, *J* = 2.2 Hz, 1H), 7.85 (dd, *J* = 8.7, 2.1 Hz, 1H), 7.78 (td, *J* = 8.6, 6.1 Hz, 1H), 7.58 (d, *J* = 8.0 Hz, 2H), 7.52 (d, *J* = 8.1 Hz, 2H), 7.38 (d, *J* = 4.4 Hz, 1H), 6.94-6.89 (m, 1H), 6.87 - 6.82 (m, 1H), 3.94 (s, 3H), 3.80 (s, 2H), 2.68 (s, 4H), 1.86 (s, 4H). |
| **13** | 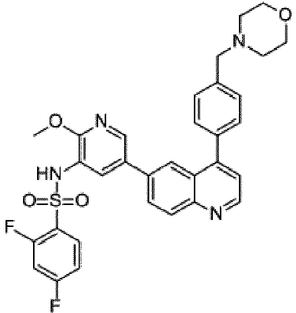 | ¹H NMR (300 MHz, CDCl₃) δ 8.95 (d, *J*= *4.4* Hz, 1H), 8.25 (d, *J* = 8.8 Hz, 1H), 8.11 (d, *J* = 2.3 Hz, 1H), 8.02 (d, *J* = 2.0 Hz, 1H), 7.98 (d, *J* = 2.3 Hz, 1H), 7.86 (dd, *J* = 8.8, 2.0 Hz, 1H), 7.82 - 7.73 (m, 2H), 7.59 - 7.53 (m, 4H), 7.38 (d, *J* = 4.5 Hz, 1H), 6.97 - 6.88 (m, 1H), 6.88 - 6.80 (m, 1H), 3.94 (s, 3H), 3.76 (s, 4H), 3.65 (s, 2H), 2.57 (s, 4H). |
| **14** | 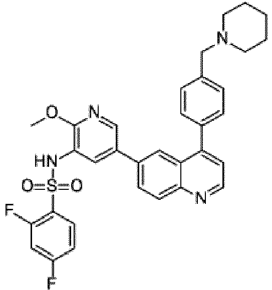 | ¹H NMR (600 MHz, CDCl₃) δ 8.95 (d, *J* = 4.4 Hz, 1H), 8.24 (d, *J* = 8.7 Hz, 1H), 8.11 (d, *J* = 2.2 Hz, 1H), 8.02 (d, *J* = 1.8 Hz, 1H), 7.98 (d, *J* = 2.2 Hz, 1H), 7.86 (dd, *J* = 8.7, 2.0 Hz, 1H), 7.77 (td, *J* = 8.4, 6.0 Hz, 1H), 7.58 (d, *J* = 7.6 Hz, 2H), 7.52 (d, *J* = 7.6 Hz, 2H), 7.38 (d, *J* = 4.4 Hz, 1H), 6.94-6.89 (m, 1H), 6.82 - 6.87 (m, 1H), 3.95 (s, 3H), 3.67 (s, 2H), 2.50 (s, 4H), 1.65 (s, 4H), 1.49 (s, 2H). |
| **15** | 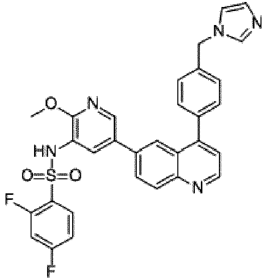 | ¹H NMR (600 MHz, CDCl₃) δ 8.96 (d, *J* = 4.4 Hz, 1H), 8.25 (d, *J* = 8.7 Hz, 1H), 8.09 (d, *J* = 2.2 Hz, 1H), 7.98 (d, *J* = 2.2 Hz, 1H), 7.94 (d, *J* = 1.9 Hz, 1H), 7.86 (dd, *J* = 8.7, 2.1 Hz, 1H), 7.76 (td, *J* = 8.4, 6.1 Hz, 1H), 7.68 (s, 1H), 7.55 (d, *J* = 8.1 Hz, 2H), 7.38 - 7.33 (m, 3H), 7.14 (s, 1H), 7.03 (s, 1H), 6.94 - 6.89 (m, 1H), 6.86 - 6.81 (m, 1H), 5.27 (s, 2H), 3.93 (s, 3H). |
| **16** | 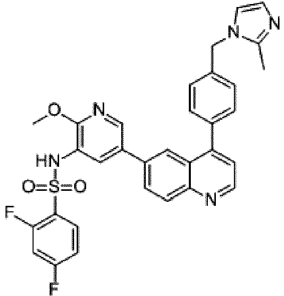 | ¹H NMR (600 MHz, CDCl₃) δ 8.95 (d, *J* = 4.4 Hz, 1H), 8.25 (d, *J* = 8.7 Hz, 1H), 8.09 (d, *J* = 2.2 Hz, 1H), 7.98 (d, *J* = 2.2 Hz, 1H), 7.95 (d, *J* = 1.9 Hz, 1H), 7.86 (dd, *J* = 8.7, 2.1 Hz, 1H), 7.76 (td, *J* = 8.4, 6.0 Hz, 1H), 7.53 (d, *J* = 8.1 Hz, 2H), 7.35 (d, *J* = 4.4 Hz, 1H), 7.26 (s, 1H), 7.25 (s, 1H), 7.00 (s, 1H), 6.94 (s, 1H), 6.93 - 6.90 (m, 1H), 6.85 - 6.80 (m, 1H), 5.19 (s, 2H), 3.93 (s, 3H), 2.42 (s, 3H). |
| **17** | 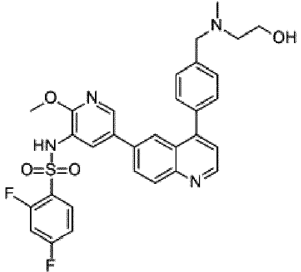 | ¹H NMR (600 MHz, CDCl₃) δ 8.96 (d, *J* = 4.4 Hz, 1H), 8.25 (d, *J* = 8.7 Hz, 1H), 8.11 (d, *J* = 2.3 Hz, 1H), 8.01 (d, *J* = 2.0 Hz, 1H), 7.98 (d, *J* = 2.3 Hz, 1H), 7.86 (dd, *J* = 8.7, 2.1 Hz, 1H), 7.78 (td, *J* = 8.5, 6.0 Hz, 1H), 7.53 (s, 4H), 7.38 (d, J= 4.4 Hz, 1H), 6.94 - 6.89 (m, 1H), 6.86 - 6.81 (m, 1H), 3.94 (s, 3H), 3.71 (s, 2H), 3.69 (t, *J* = 5.3 Hz, 2H), 2.70 (t, J= 5.3 Hz, 2H), 2.35 (s, 3H). |
| **18** | 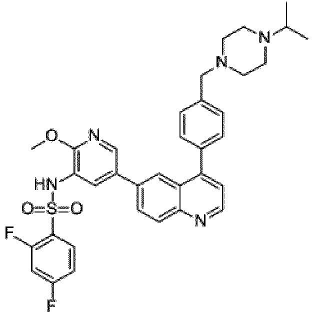 | ¹H NMR (600 MHz, CDCl₃) δ 8.94 (d, *J* = 4.3 Hz, 1H), 8.23 (d, *J* = 8.7 Hz, 1H), 8.10 (d, *J* = 2.0 Hz, 1H), 8.02 (d, *J* = 1.2 Hz, 1H), 7.98 (d, *J* = 2.0 Hz, 1H), 7.85 (dd, *J* = 8.7, 1.7 Hz, 1H), 7.76 (m, 1H), 7.53 (d, *J* = 7.8 Hz, 2H), 7.50 (d, *J* = 8.1 Hz, 2H), 7.36 (d, J= 4.4 Hz, 1H), 6.93 - 6.88 (m, 1H), 6.85 - 6.80 (m, 1H), 3.93 (s, 3H), 3.64 (s, 2H), 2.76 (s, 1H), 2.66 (s, 8H), 1.10 (d, J= 6.1 Hz, 6H). |
| **19** | 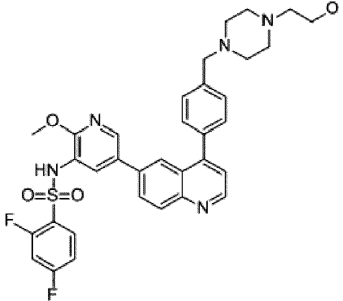 | ¹H NMR (600 MHz, CDCl₃) δ 8.95 (d, *J* = 4.4 Hz, 1H), 8.24 (d, *J* = 8.7 Hz, 1H), 8.11 (d*, J* = 2.2 Hz, 1H), 8.02 (d, *J* = 1.9 Hz, 1H), 7.98 (d, *J* = 2.2 Hz, 1H), 7.86 (dd, *J* = 8.7, 2.0 Hz, 1H), 7.77 (td, *J* = 8.5, 6.1 Hz, 1H), 7.54 (d, *J* = 8.2 Hz, 2H), 7.51 (d, *J* = 8.2 Hz, 2H), 7.37 (d, *J* = 4.4 Hz, 1H), 6.94-6.89 (m, 1H), 6.86 - 6.81 (m, 1H), 3.94 (s, 3H), 3.65 - 3.61 (m, 5H), 2.62 - 2.53 (m, 10H). |
| **20** | 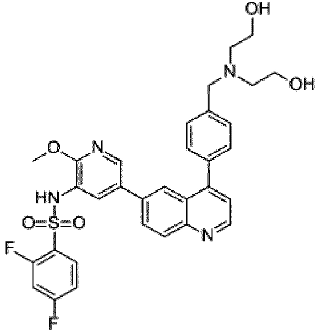 | ¹H NMR (600 MHz, CDCl₃) δ 8.94 (d, *J* = 4.2 Hz, 1H), 8.24 (d, *J* = 8.6 Hz, 1H), 8.11 (s, 1H), 7.99 (s, 1H), 7.96(s, 1H), 7.86 (d, *J* = 8.4 Hz, 1H), 7.77 (dd, *J* = 14.4, 8.1 Hz, 1H), 7.56 (d, *J* = 7.6 Hz, 2H), 7.51 (d, *J* = 7.6 Hz, 2H), 7.37 (d, *J* = 4.2 Hz, 1H), 6.91 (t*, J* = 8.6 Hz, 1H), 6.84 (t*, J* = 8.0 Hz, 1H), 3.92 (s, 3H), 3.87 (s, 2H), 3.72 (t, *J* = 4.7 Hz, 4H), 3.64 (s, 2H), 2.83 (t, *J* = 4.8 Hz, 4H). |
| **21** | 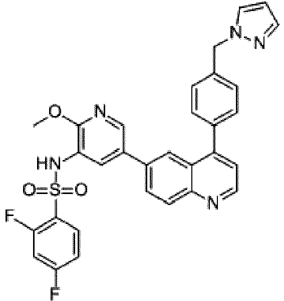 | ¹H NMR (600 MHz, CDCl₃) δ 8.94 (d, *J* = 4.4 Hz, 1H), 8.24 (d, *J* = 8.7 Hz, 1H), 8.08 (d, *J* = 2.2 Hz, 1H), 7.97 (d, *J* = 2.2 Hz, 1H), 7.95 (d, *J* = 2.0 Hz, 1H), 7.85 (dd, *J* = 8.7, 2.1 Hz, 1H), 7.73 (td, *J* = 8.5, 6.0 Hz, 1H), 7.57 (d, *J* = 1.6 Hz, 1H), 7.53 (t, *J* = 5.0 Hz, 3H), 7.40 (d, *J* = 8.1 Hz, 2H), 7.34 (d, *J* = 4.4 Hz, 1H), 6.94 - 6.89 (m, 1H), 6.82 - 6.77 (m, 1H), 6.31 (t, *J* = 2.1 Hz, 1H), 5.46 (s, 2H), 3.94 (s, 3H). |
| **22** | 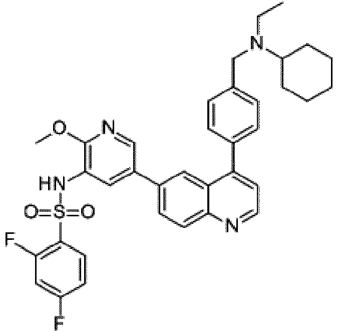 | ¹H NMR (600 MHz, CDCl₃) δ 8.94 (d, *J* = 4.4 Hz, 1H), 8.24 (d, *J* = 8.7 Hz, 1H), 8.10 (d, *J* = 2.2 Hz, 1H), 8.05 (d, *J* = 1.8 Hz, 1H), 7.98 (d, *J* = 2.1 Hz, 1H), 7.85 (dd, *J* = 8.7, 2.0 Hz, 1H), 7.78 (td, *J* = 8.5, 6.1 Hz, 1H), 7.58 (d, *J* = 7.9 Hz, 2H), 7.49 (d, *J* = 8.0 Hz, 2H), 7.39 (d, *J* = 4.4 Hz, 1H), 6.95-6.88 (m, 1H), 6.82-6.77 (m, 1H), 3.94 (s, 3H), 3.74 (s, 2H), 2.63 - 2.54 (m, 3H), 1.88 (d*, J* = 12.1 Hz, 2H), 1.80 (d, *J* = 12.7 Hz, 2H), 1.63 (d, *J* = 13.0 Hz, 1H), 1.26 (tt, *J* = 25.5, 12.6 Hz, 4H), 1.14-1.07 (m, 1H), 1.03 (t, *J* = 7.1 Hz, 3H). |
| **23** | 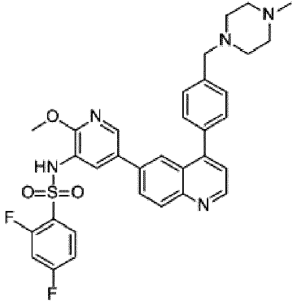 | ¹H NMR (600 MHz, CDCl₃) δ 8.95 (d, *J* = 4.4 Hz, 1H), 8.24 (d, *J* = 8.7 Hz, 1H), 8.10 (d, *J* = 2.2 Hz, 1H), 8.02 (d, *J* = 1.8 Hz, 1H), 7.98 (d, *J* = 2.2 Hz, 1H), 7.85 (dd, *J* = 8.7, 2.0 Hz, 1H), 7.77 (td, *J* = 8.4, 6.1 Hz, 1H), 7.54 (d, J= 8.1 Hz, 2H), 7.51 (d, *J* = 8.2 Hz, 2H), 7.37 (d, *J* = 4.4 Hz, 1H), 6.94-6.89 (m, 1H), 6.85 - 6.81 (m, 1H), 3.94 (s, 3H), 3.64 (s, 2H), 2.54 (s, 8H), 2.31 (s, 3H). |
| **24** | 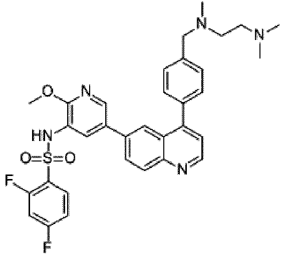 | ¹H NMR (600 MHz, CDCl₃) δ 8.93 (d, *J* = 4.4 Hz, 1H), 8.21 (d, *J* = 8.5 Hz, 1H), 8.12 (s, 1H), 8.01 (m, 2H), 7.88 (d, *J* = 6.0 Hz, 1H), 7.83 (d, *J* = 8.8 Hz, 1H), 7.57 - 7.51 (m, 4H), 7.40 - 7.37 (m, 1H), 6.90 (s, 1H), 6.74 (s, 1H), 3.90 (s, 3H), 3.55 (s, 2H), 2.30 (s, 4H), 2.24 (s, 9H). |
| **25** | 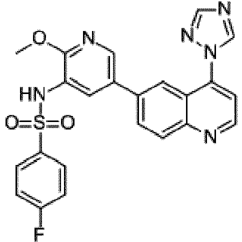 | ¹H NMR (600 MHz, CDCl₃) δ 9.08 (d, *J= 4.4* Hz, 1H), 8.65 (s, 1H), 8.39 - 8.32 (m, 2H), 8.24 (d, *J* = 1.7 Hz, 1H), 8.19 (d, *J* = 2.3 Hz, 1H), 8.08 (d, *J* = 2.2 Hz, 1H), 8.01 (dd, *J* = 8.8, 1.9 Hz, 1H), 7.90-7.80 (m, 2H), 7.54 (d, *J* = 4.5 Hz, 1H), 7.16 (t, *J* = 8.5 Hz, 2H), 7.04 (s, 1H), 3.91 (s, 3H). |
| **26** | 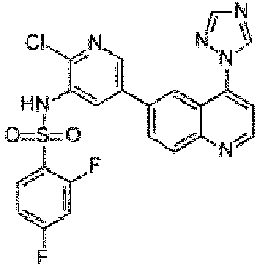 | ¹H NMR (600 MHz, CDCl₃) δ 9.12 (d, *J* = 4.6 Hz, 1H), 8.66 (s, 1H), 8.44 (d, *J* = 2.3 Hz, 1H), 8.39 (d, *J* = 8.7 Hz, 1H), 8.37 (s, 1H), 8.33 (d, *J* = 2.0 Hz, 1H), 8.26 (d, *J* = 2.3 Hz, 1H), 8.01 (dd, *J* = 8.7, 2.0 Hz, 1H), 7.94 - 7.88 (m, 1H), 7.57 (d, *J* = 4.6 Hz, 1H), 7.46 (s, 1H), 7.02 - 6.98 (m, 2H). |
| **27** | 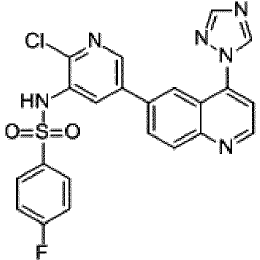 | ¹H NMR (600 MHz, DMSO) δ 9.37 (s, 1H), 9.13 (d, *J* = 4.6 Hz, 1H), 8.67 (s, 1H), 8.51 (s, 1H), 8.40 (d, *J* = 1.7 Hz, 1H), 8.32 (d, *J* = 8.9 Hz, 1H), 8.21 (dd, *J* = 8.9, 1.7 Hz, 1H), 8.06 (d, *J* = 2.2 Hz, 1H), 7.88 (d, *J* = 4.7 Hz, 1H), 7.84 (d, *J* = 5.2 Hz, 1H), 7.82 (d, *J* = 5.1 Hz, 1H), 7.42 (t, *J* = 8.7 Hz, 2H). |
| **28** | 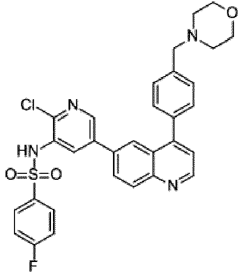 | ¹H NMR (300 MHz, CDCl₃) δ 9.01 (d, *J* = *4.4* Hz, 1H), 8.39 (d, *J* = 2.0 Hz, 1H), 8.32 (d, *J* = 8.7 Hz, 1H), 8.22 (d, *J* = 2.2 Hz, 1H), 8.13 (d, *J* = 1.7 Hz, 1H), 7.92 (dd, *J* = 8.7, 2.1 Hz, 1H), 7.78 (dd, *J* = 8.6, 4.8 Hz, 2H), 7.56 (q, *J* = 7.9 Hz, 4H), 7.43 (d, *J* = 4.5 Hz, 1H), 7.11 (t, *J* = 8.4 Hz, 2H), 3.93-3.69 (t, 4H), 3.64 (s, 2H), 2.55 (s, 4H). |
| **29** | 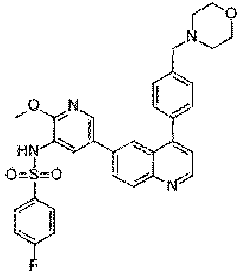 | ¹H NMR (600 MHz, CDCl₃) δ 8.98 (d, *J* = *4.4* Hz, 1H), 8.28 (d, *J* = 8.7 Hz, 1H), 8.14 (d, *J* = 2.2 Hz, 1H), 8.08 (d, *J* = 1.9 Hz, 1H), 8.05 (d, *J* = 2.2 Hz, 1H), 7.91 (dd, *J* = 8.7, 2.1 Hz, 1H), 7.81 - 7.77 (m, 2H), 7.57 (d, *J* = 8.4 Hz, 2H), 7.55 (d, *J* = 8.4 Hz, 2H), 7.40 (d, *J* = 4.4 Hz, 1H), 7.11 - 7.06 (m, 2H), 3.89 (s, 3H), 3.78 - 3.74 (m, 4H), 3.63 (s, 2H), 2.55 (s, 4H). |
| **30** | 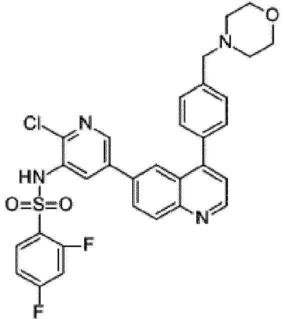 | ¹H NMR (600 MHz, CDCl₃) δ 9.01 (d, *J* = *4.4* Hz, 1H), 8.39 (d, *J* = 2.3 Hz, 1H), 8.31 (d, *J* = 8.7 Hz, 1H), 8.20 (d, *J* = 2.3 Hz, 1H), 8.11 (d, *J* = 2.0 Hz, 1H), 7.90 (dd, *J* = 8.7, 2.1 Hz, 1H), 7.79 (dd, *J* = 14.5, 8.4 Hz, 1H), 7.59 (d, *J* = 7.6 Hz, 2H), 7.54 (d, *J* = 8.0 Hz, 2H), 7.43 (d, *J* = 4.4 Hz, 1H), 6.97 (ddd, *J* = 10.5, 8.4, 2.4 Hz, 1H), 6.89 (td, *J* = 8.9, 2.4 Hz, 1H), 3.81 - 3.74 (m, 4H), 3.65 (s, 2H), 2.56 (s, 4H). |
| **31** | 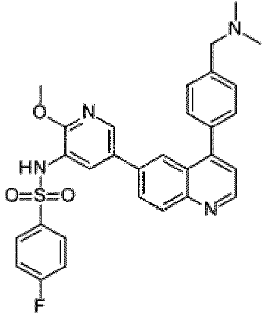 | ¹H NMR (600 MHz, DMSO) δ 8.96 (d, J= 4.4 Hz, 1H), 8.24 (d, *J* = 2.3 Hz, 1H), 8.20 (d, *J* = 8.7 Hz, 1H), 8.04 (dd, *J* = 8.7, 2.1 Hz, 1H), 8.01 (d, *J* = 2.0 Hz, 1H), 7.81 (d, *J* = 2.3 Hz, 1H), 7.77 (m, 5.2 Hz, 2H), 7.63 (d, *J* = 8.2 Hz, 2H), 7.55 (d, *J* = 8.2 Hz, 2H), 7.52 (d, *J* = 4.4 Hz, 1H), 7.34 (t, *J* = 8.8 Hz, 2H), 3.70 (s, 3H), 3.57 (s, 2H), 2.25 (s, 6H). |
| **32** | 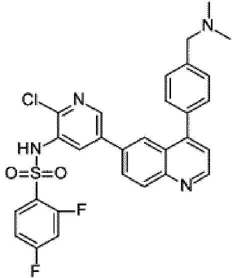 | ¹H NMR (600 MHz, DMSO) δ 8.98 (d, *J* = 4.4 Hz, 1H), 8.20 (d, *J* = 9.0 Hz, 1H), 7.98 (d, *J* = 8.1 Hz, 2H), 7.95 (s, 1H), 7.78 (s, 1H), 7.73 (q, *J* = 8.1 Hz, 5H), 7.52 (d, *J* = 4.3 Hz, 1H), 7.22 (t, *J* = 9.0 Hz, 1H), 6.97 (t, *J* = 7.4 Hz, 1H), 4.31 (s, 2H), 2.75 (s, 6H). |
| **33** | 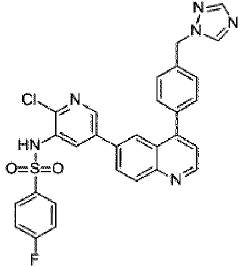 | ¹H NMR (600 MHz, DMSO) δ 9.00 (d, *J* = 4.4 Hz, 1H), 8.74 (s, 1H), 8.59 (d, *J* = 2.3 Hz, 1H), 8.25 (d, *J* = 8.6 Hz, 1H), 8.09 (dd, *J* = 8.7, 2.1 Hz, 1H), 8.06 (d, *J* = 1.9 Hz, 1H), 8.01 (s, 1H), 7.94 (d, *J* = 2.3 Hz, 1H), 7.77 (dd, *J* = 8.9, 5.1 Hz, 2H), 7.68 (d, *J* = 8.3 Hz, 2H), 7.53 (d, *J* = 4.4 Hz, 1H), 7.52 (d, *J* = 8.4 Hz, 2H), 7.36 (t, *J* = 8.8 Hz, 2H), 5.58 (s, 2H). |
| **34** | 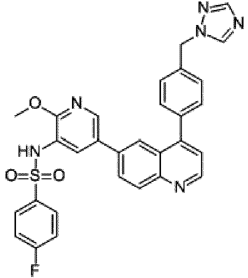 | ¹H NMR (600 MHz, CDCl₃) δ 8.98 (d, *J* = 4.4 Hz, 1H), 8.29 (d, *J* = 8.6 Hz, 1H), 8.21 (s, 1H), 8.11 (d, *J* = 2.2 Hz, 1H), 8.04 (d, *J* = 2.3 Hz, 1H), 8.02 (s, 1H), 7.98 (d, *J* = 1.9 Hz, 1H), 7.91 (dd, *J* = 8.7, 2.1 Hz, 1H), 7.78 (m, *J* = 9.0, 5.0 Hz, 2H), 7.60 (d, *J* = 8.2 Hz, 2H), 7.49 (d, *J* = 8.3 Hz, 2H), 7.38 (d, *J* = 4.4 Hz, 1H), 7.08 (t, *J* = 8.6 Hz, 2H), 5.50 (s, 2H), 3.88 (s, 3H). |
| **35** | 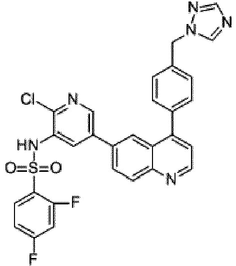 | ¹H NMR (600 MHz, CDCl₃) δ 9.02 (d, *J* = 4.5 Hz, 1H), 8.37 (d, *J* = 2.2 Hz, 1H), 8.33 (d, *J* = 8.5 Hz, 1H), 8.23 (s, 1H), 8.19 (d, *J* = 2.3 Hz, 1H), 8.03 (s, 1H), 8.02 (d, *J* = 1.9 Hz, 1H), 7.90 (dd, *J* = 8.7, 2.0 Hz, 1H), 7.77 (m, 1H), 7.59 (d, *J* = 8.2 Hz, 2H), 7.50 (d, *J* = 8.0 Hz, 2H), 7.42 (d, *J* = 4.4 Hz, 1H), 6.97 (ddd, *J* = 10.7, 8.3, 2.5 Hz, 1H), 6.88 (td, *J* = 9.0, 2.8 Hz, 1H), 5.51 (s, 2H). |
| **36** | 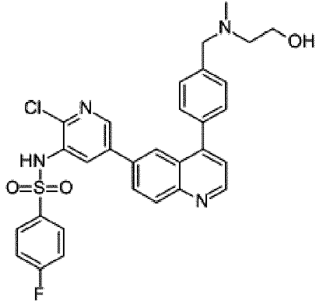 | ¹H NMR (600 MHz, CDCl₃) δ 9.02 (d, *J* = 4.4 Hz, 1H), 8.39 (d, *J* = 2.2 Hz, 1H), 8.32 (d, *J* = 8.7 Hz, 1H), 8.22 (d, *J* = 2.1 Hz, 1H), 8.12 (d, *J* = 2.0 Hz, 1H), 7.93 (dd, *J* = 8.7, 2.1 Hz, 1H), 7.81 - 7.76 (m, 2H), 7.56 (m, 4H), 7.45 (d, *J* = 4.4 Hz, 1H), 7.11 (t, *J* = 8.5 Hz, 2H), 3.74 (s, 2H), 3.70 (t, *J* = 5.3 Hz, 2H), 3.51 (s, 1H), 2.71 (t, *J* = 5.3 Hz, 2H), 2.36 (s, 3H). |
| **37** | 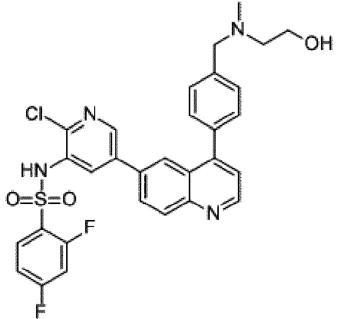 | ¹H NMR (600 MHz, CDCl₃) δ 9.02 (d, *J* = 4.4 Hz, 1H), 8.40 (d, *J* = 2.3 Hz, 1H), 8.32 (d, *J* = 8.7 Hz, 1H), 8.19 (d, *J* = 2.3 Hz, 1H), 8.08 (d, *J* = 1.8 Hz, 1H), 7.91 (dd, *J* = 8.7, 2.2 Hz, 1H), 7.83 - 7.77 (m, 1H), 7.60 (d, *J* = 8.4 Hz, 2H), 7.57 (d, *J* = 8.3 Hz, 2H), 7.45 (d, *J* = 4.4 Hz, 1H), 6.97 (ddd, *J* = 10.4, 8.1, 2.4 Hz, 1H), 6.92 - 6.87 (m, 1H), 3.85 (s, 2H), 3.78 - 3.75 (m, 2H), 2.82 - 2.78 (m, 3H), 2.46 (s, 3H). |
| **38** | 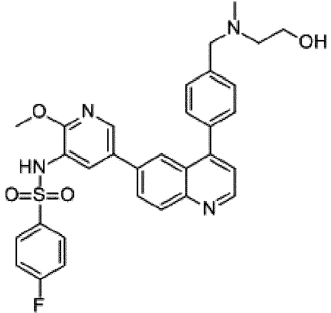 | ¹H NMR (600 MHz, CDCl₃) δ 8.99 (d, *J* = *4.4* Hz, 1H), 8.29 (d, *J* = 8.8 Hz, 1H), 8.14 (d, *J* = 2.3 Hz, 1H), 8.05 (d, *J* = 2.0 Hz, 1H), 8.04 (d, *J* = 2.3 Hz, 1H), 7.92 (dd, *J* = 8.7, 2.1 Hz, 1H), 7.81 - 7.77 (m, 2H), 7.58 (m, 4H), 7.41 (d, *J* = 4.4 Hz, 1H), 7.11-7.06 (m, 2H), 3.88(s, 3H), 3.84 (s, 2H), 3.76(t, *J* = 5.3 Hz, 2H), 2.80 (t, J=5.3 Hz, 2H), 2.45 (s, 3H). |

**Table 2 MS data and melting point of part of compound**

| Compound number | **ESI MS[M+H]⁺** | melting point (°C) | Compound number | **ESI MS[M+H]**⁺ | melting point (°C) |
|---|---|---|---|---|---|
| **1** | 495.84 | 123-124 | **20** | 621.1983* | 90-91 |
| **2** | 497.43 | 223-225 | **21** | 584.1568* | 93-95 |
| **3** | 513.1401* | 219-221 | **22** | 643.2554* | 87-88 |
| **4** | 462.56 | 171-173 | **23** | 616.2194* | 106-108 |
| **5** | 590.00 | 106-108 | **24** | 618.2350* | 87-88 |
| **6** | 518.1350* | 175-176 | **25** | 477.19 | 122-124 |
| **7** | 562.88 | 69-72 | **26** | 499.13 | 118-121 |
| **8** | 576.80 | 90-92 | **27** | 481.34 | 258-261 |
| **9** | 561.1772* | 149-150 | **28** | 589.18 | 215-216 |
| **10** | 585.1520* | 121-123 | **29** | 585.19 | 170-172 |
| **11** | 601.1723* | 115-117 | **30** | 607.18 | 165-166 |
| **12** | 587.1928* | 129-130 | **31** | 543.21 | 150-152 |
| **13** | 603.1878* | 97-99 | **32** | 565.15 | 160-164 |
| **14** | 601.2085* | 98-99 | **33** | 571.19 | 235-236 |
| **15** | 584.1568* | 108-109 | **34** | 567.19 | 150-152 |
| **16** | 598.1724* | 110-111 | **35** | 589.21 | 145-148 |
| **17** | 591.1878* | 84-85 | **36** | 577.16 | 214-217 |
| **18** | 644.2507* | 95-96 | **37** | 595.28 | 132-135 |
| **19** | 646.2300* | 96-97 | **38** | 573.40 | ND |

| | | | | | |
|---|---|---|---|---|---|
| * Data was measured by the high resolution mass spectrometry (HRMS). | | | | | |

The present invention further relates to a pharmaceutical composition, which comprises a compound in any of the above and pharmaceutically acceptable carrier. It may be in solid form or liquid form, and the pharmaceutical dosage form may be tablets, capsules, powders, granules, suspensions, or injections. When the compounds of the present invention were used for the above purposes, they may be combined with one or more pharmaceutically acceptable carriers or excipients, such as solvents, diluents and the like, and can be administered orally as follows: tablets, pills, capsules, dispersible powders, granules or suspensions (containing, for example about 0.05 to 5% suspending agent), syrups (containing, for example about 10 to 50% sugar), and elixirs (containing about 20-50% ethanol), or in a way other than administration: ointments, gels, medicated tape, etc., or in sterile injectable solutions or suspensions (containing about 0.05-5% suspending agent in an isotonic medium) non-parenteral administration. For example, the pharmaceutical preparations may contain about 0.01 to 99% of the active ingredient mixed with the carrier, more preferably about (by weight) from 0.1% to 90%.

The present invention further relates to a pharmaceutical composition, which comprises a compound defined hereinabove and an anti-tumor compound selected from the group consisting of: anti-microtubule agents, platinum coordination complexes, alkylating agents, antibiotics, topoisomerase II inhibitors, antimetabolites, topoisomerase I inhibitors, hormones and hormonal analogues, signal transduction pathway inhibitors, non-receptor tyrosine kinase angiogenesis inhibitors, immunosuppressive agents, pro-apoptotic and cell cycle inhibitors signal transduction inhibitors. Preferably, the anti-tumor compound is currently used in clinical.

Because the pharmaceutically active compounds of the present invention have the activity of PI3K inhibitor, in particular the compounds regulating/suppressing PI3Kα used for treating cancer. Because the pharmaceutically active compounds of the invention also have the activity of PI3Kα, PI3Kβ, PI3δ and PI3Kγ, they show therapeutic value in the treatment of the following diseases: autoimmune diseases, inflammatory diseases, cardiovascular diseases, neurological degenerative diseases, allergies, asthma, pancreatitis, multi-organ failure, kidney disease, platelet aggregation, sperm motility, transplantation rejection, graft removal and lung damage.

The present invention also relates to the use of compounds in any claim above for the preparation of inhibiting phosphatidylinositol 3-kinase medicine. Preferably, the drugs are anticancer drugs or immunosuppressive drugs. More preferably, the tumor is lung cancer, leukemia, colon cancer, kidney cancer, prostate cancer, melanoma, liver cancer, ovarian cancer, breast cancer or brain cancer.

The effective dose of the formula (1) compound of the present invention varies depending on the compounds used, the administration mode and the severity degree of the disease to be treated. However, satisfactory results can be obtained, when the compounds of the present invention were administered with the daily dose of about 0.25-1000 mg/kg animal weight; preferably, administered with 2-4 divided doses everyday, or administered in the form of sustained release. For most of the large mammals, the total daily dose is about 1-100 mg/kg, preferably about 2-80 mg/kg. Dosage forms suitable for oral administration contain about 0.25-500 mg of the active compounds intimately mixed with a solid or liquid pharmaceutically acceptable carrier. The dose can be adjusted to provide the optimal therapeutic response. For example, according to the urgent requirements of the treatment status, several divided doses can be administered daily or the dose can be reduced proportionally.

These active compounds may be administered through the oral, intratumor, intravenous, intramuscular or subcutaneous route, etc.. The solid carriers include: starch, lactose, dicalcium phosphate, microcrystalline cellulose, sucrose and kaolin, while liquid carriers include: sterile water, polyethylene glycol, nonionic surfactant and edible oil (such as corn oil, peanut oil and sesame oil), as long as the carriers are suitable for the properties of the active ingredient and the particular administration mode as desired. Adjuvants normally used in the preparation of pharmaceutical compositions may also advantageously be included, such as flavoring agents, pigments, preservatives and antioxidants such as vitamin E, vitamin C, BHT and BHA.

For being prepared and administrated readily, the preferred pharmaceutical composition is a solid composition, particularly tablets and solid filled or liquid filled capsules. Preferably, compounds are administrated through intratumoral and oral routes.

These active compounds may also be administrated parenterally or intraperitoneally. The solution or suspension of these active compounds (as free base or pharmaceutically acceptable salt) can be prepared in water properly containing surfactant (such as hydroxypropyl cellulose, polyvinyl pyrrolidone). Furthermore, the dispersion can be prepared in glycerol, liquid, polyethylene glycols and mixtures thereof in the oil. Under the conditions of conventional storage and use, these preparations can contain a preservative to prevent the growth of microorganisms.

The drug forms suitable for the injection include: a sterile aqueous solution or dispersion and sterile powder (for the extemporaneous preparation of sterile injectable solution or dispersion). In all cases, the forms must be sterile and fluid for being discharged from a syringe. Further, the forms must be stable under the conditions of manufacture and storage, and the pollution of the microorganisms (such as bacteria and fungi) should be prevented. The carrier can be a solvent or dispersion medium containing water, alcohol (such as glycerol, propylene glycol and liquid polyethylene glycol), appropriate mixtures thereof, and vegetable oils.

The term "safe and effective dosage" refers to the amount of the compounds which is sufficient to improve the patient's condition without producing any serious side effect. The safe and effective amount was determined according to the subject's age, conditions, course of treatment, etc..

"Pharmaceutically acceptable carrier" means one or more compatible solid or liquid fillers or gel materials, which are suitable for human, and must have sufficient purity and sufficiently low toxicity. "Compatibility" herein means that the components of the composition can be blended with the compounds of the invention or with each other, and would not significantly reduce the efficacy of the compounds. Some examples of pharmaceutically acceptable carriers include sugar (such as glucose, sucrose, lactose, etc.), starch (such as corn starch, potato starch, etc.), cellulose and the derivatives thereof (such as sodium carboxymethyl cellulose, sodium ethyl cellulose, cellulose acetate, etc.), gelatin, talc, solid lubricants (such as sodium stearate, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween), wetting agent (such as sodium dodecyl sulfate), coloring agents, flavoring agents, stabilizers, antioxidants, preservatives, pyrogen-free water, etc.

### DESCRIPTION OF FIGURES OF THE INVENTION

FIG 1 shows the structure of formula(1) of the compound of the present invention, while groups therein are as defined in the specification.
FIG 2 shows the result of the activity of compound 10.
FIG 3 shows the result of the activity of compound 17.
FIG 4 shows the result of the activity of compound 9.
FIG 5 shows average rat plasma concentration - time curve of compound 9 by tail vein injection of compound 9
FIG 6 shows average rat plasma concentration - time curve of compound 9 by gavage of compound 9.

### DETAILED DESCRIPTION OF THE INVENTION

The present invention will be further illustrated below with reference to specific examples. It should be understood that these examples are only to illustrate the present invention but not to limit the scope of the present invention. The experimental methods with no specific conditions described in the following examples are generally performed under conventional conditions or according to the manufacture's instruction. Unless indicated otherwise, the percentages, or parts are calculated by weight. Reagents used in the experiments were analytical grade from the market.

### Example 1:

### Preparation of N- (5- (4- (1H-1, 2, 4- triazole-1-yl) quinolin-6-yl) -2-methoxypyridine-3-yl) -2, 4- difluorobenzenesulfonamide (IV, compound No. 1 in the table)

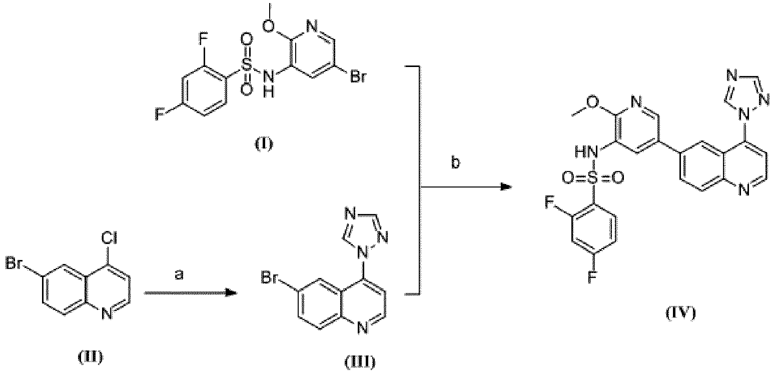

### a) Preparation of 6-bromo -4- (1H-1, 2, 4- triazole-1-yl) quinoline (III):

6-bromo-4-chloro-quinoline (0.5 g, 2.06 mmol) was added into 25ml one-necked flask, triazole (0.71 g, 10.30 mmol), was dissolved in 4 mL of 1, 4-dioxane, refluxed and stirred for 4 hours under 90°C, cooling to room temperature, chloroform and saturated sodium bicarbonate solution were added to extract, the organic layer was washed with water and saturated brine, dried with anhydrous magnesium sulfate, and evaporated to dry under reduced pressure, 0.24 g solid product was crystallized by ether , yield 42.9%. MS(ESI)m/z : 275.16 (M + H⁺, ⁷⁹Br), 277.16 (M + H⁺, ⁸¹Br).

### b) Preparation of N- (5- (4- (1H-1, 2, 4- triazole-1-yl) quinolin-6-yl) -2-methoxypyridine-3-yl) -2, 4- difluorobenzenesulfonamide (IV, compound No. 1 in the table)

Bromo -4- (1H-1, 2, 4-triazole 1-yl) quinoline **(III)** (0.20 g, 0.73 mmol) was dissolved in anhydrous dioxane (6 mL), bis (pinacolato) diboron (0.20 g, 0.80 mmol) and catalyst [1, 1-bis (diphenylphosphino) ferrocene] dichloropalladium dichloromethane complex (0.021 g, 0.03 mmol) was added, heated and refluxed with stirring for 3 hours at 100 °C under nitrogen protected condition. N- (5- bromo-2-methoxypyridine -3-yl) -2, 4-difluorobenzene sulfonyl chloride **(IV)** (0.30 g, 0.77 mmol), saturated sodium carbonate (1.3 mL) and another part of the catalyst [1, 1-bis (diphenylphosphino) ferrocene] dichloropalladium dichloromethane complex (0.021 g, 0.03 mmol) was added to the mixed system, heated and refluxed with stirring for 12 hours at 110 °C to obtain 0.15 g pale yellow solid , yield 41.7%. ¹H NMR (300 MHz, DMSO) δ 10.48 (s, 1H), 9.36 (s, 1H), 9.10 (d, J = 4.7 Hz, 1H), 8.51 (s, 1H), 8.44 (d, J = 2.3 Hz, 1H), 8.31 (d, J = 1.9 Hz, 1H), 8.27 (s, 1H), 8.20 (dd, J = 8.9, 1.9 Hz, 1H), 7.96 (d, J = 2.3 Hz, 1H), 7.86 (d, J = 4.7 Hz, 1H), 7.88-7.69 (m, 1H), 7.62-7.53 (m, 1H), 7.21 (td, J = 8.4, 2.2 Hz, 1H), 3.67 (s, 3H). MS(ESI)m/z : 495.84(M+H⁺).

### Examples 2-3:

Compound 2 and 3 of Table 1 may be obtained by using corresponding raw materials, with reference to the general procedure of the compound obtained in Example 1.

### Example 4:

### N- (5- (4- chloro-quinolin-6-yl) -2-methoxy-3-yl) -2, 4-difluorobenzene sulfonamide (IV, Compound No. 4 in the table)

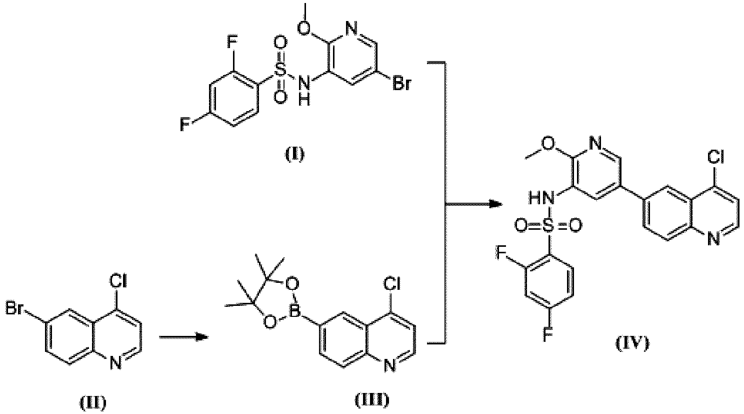

### a) Preparation of 4-chloro-6- (4, 4, 5, 5-tetramethyl-1, 3, 2-dioxaborolan-2-yl) quinoline (III):

Material **(II)** (0.50 g, 2.06 mmol) was added into 50 ml eggplant-shaped flask, and dissolved in 13 mL dioxane, bis (pinacolato) diboron (0.79 g, 3.10 mmol), potassium acetate (0.81 g, 8.25 mmol) and palladium catalyst PdCl₂ (dppf) CH₂Cl₂ (0.128 g, 0.157 mmol) were added to the system, heated and refluxed with stirring for 3 hours at 90 °C under nitrogen protected condition. After cooled to room temperature, the solvent was evaporated to dry, and the mixture was extracted with ethyl acetate (20 mL) and water (20 mL). The aqueous layer was extracted with a small amount of ethyl acetate (15 mL × 2), the organic layers were combined, dried with anhydrous sodium sulfate, and concentrated to obtain the crude product 1.15g (PH = 6), purified by silica gel column chromatography (gradient elution with dichloromethane in petroleum ether with the content of 30% -50%) to obtain 0.40g yellowish white solid, yield 68.7%.

### b) Preparation of N- (5- (4- chloro-quinolin-6-yl) -2-methoxypyridine-3-yl) -2, 4-difluorobenzene sulfonamide (IV)

Raw material **(III)** (0.40 g, 1.38 mmol) was dissolved (12 mL) in dioxane in 50 ml eggplant-shaped flask, raw material (I) (0.526 g, 1.38 mmol), palladium catalyst PdCl₂ (dppf) CH₂Cl₂ (0.056 g, 0.069 mmol) and potassium carbonate solution (4 mL) were added, heated and refluxed at 110 °C under nitrogen protected condition. After cooled to room temperature, the solvent was evaporated to dry, the mixture was extracted with dichloromethane (20 mL) and water (20 mL). The aqueous layer was extracted with a small amount of dichloromethane (15 mL × 3), and a large number of precipitate turned up in the organic layer and the aqueous layer, and was filtered to obtain 0.28 g gray product.Yield 44.4%. ¹H NMR (300 MHz, CDCl₃) δ 8.82 (d, J = 4.8 Hz, 1H), 8.28 (d, J = 2.0 Hz, 1H), 8.24 (d, J = 2.2 Hz, 1H), 8.09 (d, J = 2.2 Hz, 1H), 7.93 (m, 2H), 7.58 (d, J = 4.8 Hz, 1H), 7.32 (s, 1H), 6.98 (m, 2H), 4.00 (s, 3H), MS(ESI)m/z : 462.56(M+H⁺).

### Example 5:

### N- (5- (4- (4 - ((diethylamine) methyl) phenyl) quinolin-6-yl) -2-methoxypyridine-3-yl) -2, 4-difluorobenzene sulfonamide (V, compound No. 5 in the table)

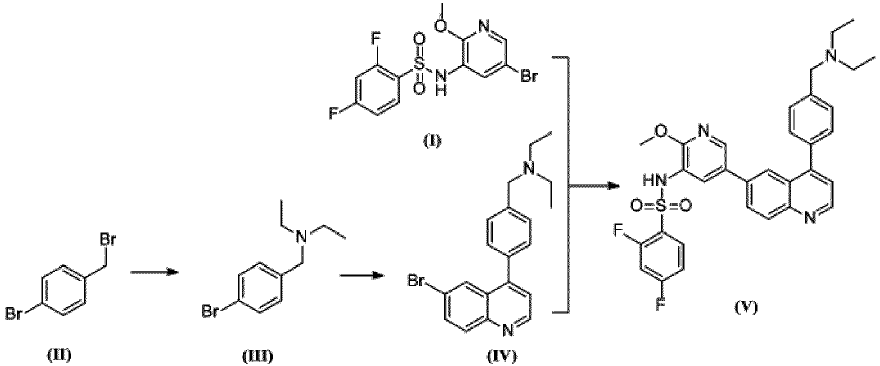

### a) Preparation of N- (4- bromobenzyl) -N- diethylamine (III)

The mixture of p-bromobenzylbromide (0.6 g, 2.4 mmol) and potassium carbonate (1.0 g, 7.2 mmol) were dissolved in 15 mL of acetonitrile, diethylamine (0.99 mL, 9.6mmol) was dropped within two minutes slowly into the mixed system, stirred for two hours at room temperature, then the mixture was concentrated under vacuum, water (15 mL) was added to dissolve, and extracted with ethyl acetate (15 mL × 3). The organic layers were combined, washed with brine, dryed with anhydrous sodium sulfate, and concentrated in vacuum to obtain 0.56 g product, yield 96.6%.

### b) Preparation of N- (4- (6- bromo-quinolin-4-yl) phenyl) -N- ethylenediamine (IV)

N- (4- bromobenzyl) -N- diethylamine (0.56 g, 2.31 mmol) was dissolved in dioxane (15 mL), bis (pinacolato) diboron was added (0.647g, 2.55 mmol), potassium acetate (0.452 g, 4.62 mmol) and palladium catalyst PdCl₂ (dppf) CH₂Cl₂ (0.057 g, 0.069 mmol) were added, heated and refluxed for 3h at 110°C under nitrogen protected condition, the reaction was stopped after detected that the raw material point had disappeared. 6-bromo-4-iodo quinoline (0.694g, 2.079mmol) and another part of palladium catalyst PdCl₂ (dppf) CH₂Cl₂ (0.057 g, 0.069 mmol) and a solution of 1M K₂CO₃ (5.00 mL) were added to the mixed system, heated and refluxed for 10h at 110°C under nitrogen protected condition and then the reaction was stopped. The solvent was evaporated to dry, and water (50 mL) was added, and extracted with dichloromethane (4 × 15 mL), the organic layers were combined, washed with brine (15 mL), dried with anhydrous magnesium sulfate. The solvent was evaporated under reduced pressure, purified by silica gel column chromatography (eluent: dichloromethane / methanol = 20: 1) to obtain 0.36 g product, yield 42.35%. MS(ESI)m/z : 369.71 (M + H⁺, ⁷⁹Br), 371.71 (M + H⁺, ⁸¹Br).

### c) Preparation of N- (5- (4- (4 - ((diethylamine) methyl) phenyl) quinolin-6-yl) -2 -methoxypyridine-3-yl) -2, 4-difluorobenzene sulfonamide (V, compound No. 5 in the table)

N- (4- (6- bromo-quinolin-4-yl) phenyl) -N- ethylenediamine (0.36 g, 1.00 mmol) was dissolved in dioxane (15 mL), bis (pinacolato) diboron (0.28 g, 1.10 mmol), potassium acetate (0.30 g, 3.00 mmol) and palladium catalyst PdCl₂ (dppf) CH₂Cl₂ (0.024 g, 0.03 mmol) was added, heated and refluxed for 3h at 100 °C under nitrogen protected condition, the reaction was stopped after detected that raw materials point had disappeared. N- (5- bromo-2- methoxypyridine -3-yl) -2, 4-difluorobenzene sulfonyl chloride (**IV**) (0.37 g, 0.96 mmol), another part of palladium catalyst PdCl₂ (dppf) CH₂Cl₂ (0.024 g, 0.03 mmol) and 1M K₂CO₃ solution (3.00 mL) were added to the mixed system, heated and refluxed for 10h at 110°C under nitrogen protected condition and then the reaction was stopped. The solvent was evaporated to dry, and water (50 mL) was added, and extracted with dichloromethane (4×5 mL), the organic layers were combined, washed with brine (15 mL), and dried with anhydrous magnesium sulfate, the solvent was evaporated under reduced pressure, and purified by silica gel column chromatography (eluent: dichloromethane / methanol = 20: 1) to obtain 0.17 g product, yield 26.2%.¹H NMR (300 MHz, DMSO) δ 8.96 (d, J = 4.5 Hz, 1H), 8.28 - 8.16 (m, 2H), 8.09 - 7.99 (m, 2H), 7.81 (d, J = 2.1 Hz, 1H), 7.76 - 7.55 (m, 6H), 7.52 (d, J = 4.1 Hz, 1H), 7.12 (t, J = 8.7 Hz, 1H), 3.84 (s, 2H), 3.68 (s, 3H), 2.67 (q, J = 7.1 Hz, 4H), 1.07 (t, J = 7.1 Hz, 6H), MS(ESI)m/z : 590.00(M+H⁺).

### Example 6

### N- (2- methoxy-5- (4- (p-tolyl) quinolin-6-yl) pyridin-3-yl) -2, 4-difluorobenzene sulfonamide (Compound No. 6 in the table)

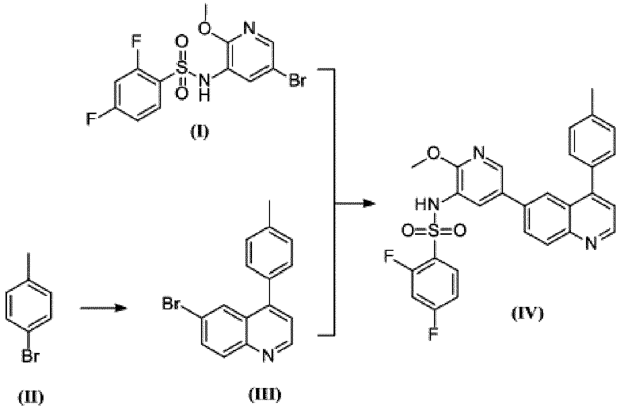

### a) Preparation of 6-bromo-4- (p-tolyl) quinoline (III)

P-bromotoluene (0.5 g, 2.92 mmol) was dissolved in dioxane (20 mL) in 50 ml eggplant-shaped flask, bis (pinacolato) diboron (0.80 g, 3.21 mmol), potassium acetate (0.57 g , 5.84 mmol) and palladium catalyst PdCl₂ (dppf) CH₂Cl₂ 0.07 g, 0.09 mmol) was added, heating and reflux for 3h at 100°C under nitrogen protected condition, the reaction was stopped after detected that raw material point had disappeared. 6-bromo-4-iodo quinoline (1.03g, 3.07mmol), another part of palladium catalyst PdCl₂ (dppf) CH₂Cl₂ (0.007 g, 0.09 mmol) and 1M K₂CO₃ (5.20 mL) solution were added to the mixed system, heated and refluxed for 10h at 110 °C under nitrogen protected condition and the reaction was stopped. The solvent was evaporated to dry, and purified by silica gel column chromatography (Petroleum ether / ethyl acetate = 10: 1) to obtain 0.36 g product, yield 41.4%.

### b) Preparation of N- (2- methoxy -5- (4- (p- tolyl) quinolin-6-yl) pyridin-3-yl) -2, 4-difluorobenzene sulfonamide (IV, table Compound No. 6)

6-bromo-4- (p-tolyl) quinoline **(III)** (0.36 g, 1.21 mmol) was dissolved in dioxane (6 mL), bis (pinacolato) diboron was added (0.34 g, 1.33 mmol), potassium acetate (0.36 g, 3.63 mmol) and palladium catalyst PdCl₂ (dppf) CH₂Cl₂ (0.05g, 0.06 mmol) were added, heated and refluxed for 3h at 100 °C under nitrogen protected condition, the reaction was stopped after detected that raw materials point had disappeared. N- (5- bromo-2-methoxypyridine -3-yl) -2, 4-difluorobenzene sulfonyl chloride **(I)** (0.46 g, 1.21 mmol), another portion of palladium catalyst PdCl₂ (dppf) CH₂Cl₂ (0.05g, 0.06 mmol) and a 1M K₂CO₃ solution (2.00 mL) were added to the mixed system, heated and refluxed for 10h at 110 °C under nitrogen protected condition and the reaction was stopped. After filtered through Celite, the solvent was evaporated to dry and purified by silica gel column chromatography (eluent: Petroleum ether / ethyl acetate = 4: 1) to obtain 0.11 g product , yield 16.5%. ¹HNMR (300 MHz, DMSO) δ 10.35 (s, 1H), 8.94 (d, J = 4.4 Hz, 1H), 8.31 (m, 1H), 8.19 (d, J = 8.7 Hz, 1H), 8.03 (m, 2H), 7.83 (m, 1H), 7.70 (dd, J = 15.5, 8.3 Hz, 1H), 7.58-7.40 (m, 6H), 7.14 (m , 1H), 3.65 (s, 3H), 2.43 (s, 3H), MS(ESI)m/z : 518.14(M+H⁺).

### Example 7

### 4- (6- (5- (2, 4-difluorobenzene sulfonamido) -6-methoxypyridine-3-yl) quinolin-4-yl) benzoic acid ethyl ester (Compound No. 8 in Table)

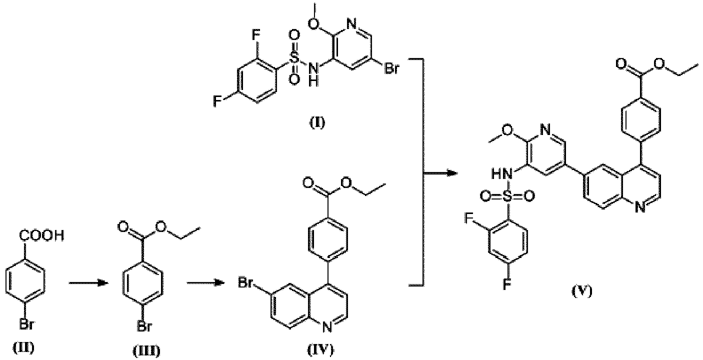

### a) Preparation of Bromobenzoate (III):

P-bromo-benzoic acid (0.5 g, 2.5 mmol) was added into 100ml round bottom flask and dissolved in 30ml ethanol, a few drops of concentrated sulfuric acid was added dropwise. Reflux with stirring at 80 °C for 12 hours. Cooled to room temperature, the solvent was evaporated to dry, dissolved in a small amount of ethyl acetate, and washed with saturated brine (× 2), dried with magnesium sulfate, and evaporated to dry under reduced pressure to obtain 0.5 g product, yield 87.4%.

### b) Preparation of 4- (6-bromo-quinolin-4-yl) benzoate (IV)

Bromobenzoate (0.53 g, 2.48 mmol) was dissolved in dioxane (20 mL), , bis (pinacolato) diboron was added (0.70 g, 2.72 mmol), potassium acetate (0.49 g, 4.96mmol) and palladium catalyst PdCl₂ (dppf) CH₂Cl₂ (0.06g, 0.074 mmol) were added, heated and refluxed for 4h at 100°C under nitrogen protected condition, the reaction was stopped after detected that raw material point had disappeared. 6-bromo-4-iodo quinoline (0.87 g, 2.60 mmol), another portion of palladium catalyst PdCl₂ (dppf) CH₂Cl₂ (0.06g, 0.074 mmol) and 1M K₂CO₃ solution (4.40 mL) were added to the mixed system, heated and refluxed for 10h at 110 °C under nitrogen protected condition then the reaction was stopped. The solvent was evaporated to dry, water (60 mL) was added, and was extracted with dichloromethane (4×20 mL), the organic layers were combined, washed with brine (20 mL), dried with anhydrous magnesium sulfate. The solvent was evaporated to dry under reduced pressure. Purified by silica gel column chromatography (eluent: petroleum ether / ethyl acetate = 5: 1) to obtain 0.46 g product, yield 54.4%.

### c) Preparation of 4- (6- (5- (2, 4-difluorobenzene sulfonamido) -6-methoxypyridine-3-yl) quinolin-4-yl) benzoic acid ethyl ester (V, Compound No. 8 in Table)

4- (6-bromo-quinolin-4-yl) benzoic acid ethyl ester (0.34 g, 1.00 mmol) was dissolved in dioxane (10 mL), bis (pinacolato) diboron (0.28 g, 1.10 mmol), potassium acetate (0.30 g, 3.00mmol) and palladium catalyst PdCl₂ (dppf) CH₂Cl₂ (0.045g, 0.05 mmol) were added, heated and refluxed for 3h at 100°C under nitrogen protected condition, the reaction was stopped after detected that raw material point had disappeared. N- (5- bromo-2-methoxypyridine -3-yl) -2, 4-difluorobenzene sulfonyl chloride (IV) (0.40 g, 1.00 mmol), another portion of palladium catalyst PdCl₂ (dppf) CH₂Cl₂ (0.045g, 0.05 mmol) and 1M K₂CO₃ solution (3.00 mL) were added to the mixed system, heated and refluxed for 10h at 110 °C under nitrogen protected condition and the reaction was stopped. The solvent was evaporated to dryness, adding water (50 mL), and extracted with dichloromethane (4×15mL), the organic layers were combined, washed with brine (15 mL), dried with anhydrous magnesium sulfate. The solvent was evaporated to dry under reduced pressure. Purified by silica gel column chromatography (eluent: petroleum ether / ethyl acetate = 4: 1) to obtain 210mg product, yield 37.5%. ¹H NMR (300 MHz, CDCl₃) δ 9.00 (d, J = 4.3 Hz, 1H), 8.33 (d, J = 9.0 Hz, 1H), 8.27 (d, J = 8.2 Hz, 2H), 8.07 (d, J = 2.2 Hz, 1H), 7.97 (d, J = 2.2 Hz, 1H), 7.90 (dd, J = 6.3, 1.9 Hz, 2H), 7.78 (td, J = 8.4, 5.8 Hz, 1H), 7.64 (d, J = 8.2 Hz, 2H), 7.44 (d, J = 4.3 Hz, 1H), 6.91 (m, 2H), 4.46 (q, J = 7.1 Hz, 2H), 3.95 (s, 3H), 1.45 (t, J = 6.7 Hz, 3H). MS(ESI)m/z : 576.80(M+H⁺).

### Example 8:

Compound 7 of Table 1 was obtained using corresponding raw materials, with reference to the general procedure of the compound obtained in Example 7.

### Examples 9-10:

Compounds 9 and 10 of Table 1 were obtained using corresponding raw materials, with reference to the general procedure of the compound obtained in Example 5.

### Example 11:

Compound 11 of Table 1 was obtained using corresponding raw materials, with reference to the general procedure of the compound obtained in Example 7.

### Examples 11-24:

Compounds 12-24 of Table 1 were obtained using corresponding raw materials, with reference to the general procedure of the compound obtained in Example 5.

### Examples 25-27:

Compounds 25-27 of Table 1 were obtained using corresponding raw materials, with reference to the general procedure of the compound obtained in Example 1.

### Examples 28-38:

Compounds 28-38 of Table 1 were obtained using corresponding raw materials, with reference to the general procedure of the compound obtained in Example 5.

### Example 39: biological measurement in the inhibiting enzyme activity of the compounds of the series:

Three methods were taken to measure the inhibiting enzyme activity of the above compounds:
Lance Ultra Assay method was used to measure IC₅₀ value of mTOR.
ADP-Glo Luminescent Kinase Assay method was used to measure IC₅₀ value of PI3Kβ and PI3Kγ.
Kinase-Glo Luminescent Kinase Assay method was used to measure IC₅₀ value of PI3Kα and PI3Kδ.

**Table 3 IC₅₀ (nM) of part of compounds on PI3Kα**

| **Compound number** | **IC₅₀ on PI3Kα** | **Compound number** | **IC₅₀ on PI3Kα** |
|---|---|---|---|
| **1** | 0.55 | **20** | 1.3 |
| **2** | >100 | **21** | 1.1 |
| **3** | 1.4 | **22** | 1.6 |
| **4** | 2.3 | **23** | 1.3 |
| **5** | 1.1 | **24** | 2.5 |
| **6** | 4.5 | **25** | 1.2 |
| **7** | 1.5 | **26** | 0.59 |
| **8** | 2.6 | **27** | 0.81 |
| **9** | 1.3 | **28** | 1.5 |
| **10** | 0.65 | **29** | 1.6 |
| **11** | 0.70 | **30** | 1.1 |
| **12** | 1.4 | **31** | 2.1 |
| **13** | 1.1 | **32** | 0.69 |
| **14** | 1.2 | **33** | 0.73 |
| **15** | 1.1 | **34** | 0.91 |
| **16** | 0.95 | **35** | 0.82 |
| **17** | 0.59 | **36** | 1.0 |
| **18** | 1.7 | **37** | 0.75 |
| **19** | 1.2 | **38** | 4.8 |
| **PI103** | 6.78 | **GSK2126458** | 0.77 |

As can be seen from Table 3, all the synthesized compounds have good inhibitory activity on PI3Kα, wherein the best is compound 1, which is 0.55 nM. On this basis, inhibitory activity against different subtypes of mTOR and PI3K of the four preferred compounds with good activity was tested, which shows better activity against individual isoform (Table 4).

**Table 4 Results of the activity of some compounds mTOR and PI3K isoforms**

| **Cpd_ID** | IC₅₀ on PI3K gamma (nM) | IC₅₀ on PI3K beta (nM) | IC₅₀ on PI3K delta (nM) | IC₅₀ on mTOR (nM) |
|---|---|---|---|---|
| **1** | 0.47 | 3.6 | 2.6 | 1.1 |
| **9** | 1.5 | 1.8 | 3.8 | 3.8 |
| **10** | 0.74 | 1.7 | 2.8 | 1.1 |
| **17** | 1.1 | 1.1 | 3.4 | 2.2 |
| **GSK2126458** | 0.61 | 1.2 | 2.5 | 0.31 |
| **PI103** | 18 | 12 | 9.4 | 8.8 |

### Example 40: Studies of in vitro anti-tumor activity of the compounds of the series:

### (1) Experimental method

1. Sample preparation: After dissolved with DMSO (Merck), PBS (-) was added to make 1000µg / ml solution or a uniform suspension, and then diluted with PBS (-) containing the DMSO. Doxorubicin (DOX) was used as the positive control drug.
2. Cell line
   1) A549 (human lung cancer cells); 2) HCT116 (human colon cancer cells); 3) HepG2 (human hepatoma cells); 4) ZR-75-30 (human breast cancer cells); 5) U87MG (human brain star glioblastoma)
3. Broth
   DMEM + 5-10% FBS + double resistant
4. Other materials
   Automatic microplate reader: Model: WellscanMK-2, Manufacturer: Labsystems
   Import 96-well culture plate, etc.
5. Experimental method
   MTT assay. 100µl cell suspension was added at a concentration of 4 ∼ 5 × 10⁴ cells / ml to each well of a 96-well plate, and was set at 37°C, 5% CO₂ in the incubator. After 24h, the sample solution was added, 10µl / hole, double wells were set, 37°C, acting for 72h at 5% CO₂. 20µl MTT solution of 5mg / ml was added to each well, the dissolving liquid was added after acted for 4h, 100µl / hole, kept in the incubator, and read 570nm OD with MK-2 automatic microplate reader after dissolution.

### (2)Test result: (see Table 5)

**Table 5 In vitro proliferation inhibition to five human tumor cells of part of compounds**

| **Compound No.** | A549 IC₅₀(µg/ml) | HCT116 IC₅₀(µg/ml) | U87MG IC₅₀(µg/ml) | HepG2 IC₅₀(µg/ml) | ZR-75-30 IC₅₀(µg/ml) |
|---|---|---|---|---|---|
| **1** | 0.00819 | 0.011 | 0.475 | 1.37 | 0.454 |
| **2** | 1.694 | 1.647 | 3.321 | 9.83 | 6.73 |
| **3** | 1.232 | 2.272 | 64.716 | 11.48 | 2.16 |
| **4** | 1.360 | 3.043 | >100 | 2.26 | 2.59 |
| **5** | 0.013 | 0.00293 | 2.877 | 0.613 | 0.783 |
| **6** | 0.642 | 1.313 | 34.699 | 5.63 | 6.69 |
| **7** | 0.014 | 0.277 | 3.902 | 1.16 | 1.74 |
| **8** | 0.043 | 0.235 | 11.712 | 2.65 | 2.45 |
| **9** | 0.00247 | 0.00169 | 0.051 | ND | ND |
| **10** | 0.00927 | 0.00507 | 0.129 | ND | ND |
| **11** | 0.048 | 0.221 | 0.284 | ND | ND |
| **12** | 0.022 | 0.011 | 0.093 | ND | ND |
| **13** | 0.042 | 0.00196 | 0.091 | ND | ND |
| **14** | 0.00934 | 0.0205 | 0.019 | ND | ND |
| **15** | 0.018 | 0.019 | 0.350 | ND | ND |
| **16** | 0.00608 | 0.00846 | 0.024 | ND | ND |
| **17** | 0.012 | 0.00280 | 0.092 | ND | ND |
| **18** | 0.00124 | 0.0159 | 0.301 | ND | ND |
| **19** | 0.016 | 0.014 | 0.137 | ND | ND |
| **20** | 0.00174 | 0.00237 | 0.089 | ND | ND |
| **21** | 0.018 | 0.031 | 0.935 | ND | ND |
| **22** | 0.013 | 0.143 | 0.930 | ND | ND |
| **23** | 0.00818 | 0.027 | 0.207 | ND | ND |
| **24** | 0.00804 | 0.032 | 0.157 | ND | ND |
| **25** | 1.02 | 1.04 | ND | 9.49 | ND |
| **26** | 2.35 | 1.23 | ND | 2.80 | ND |
| **27** | 1.16 | 1.22 | ND | 9.43 | ND |
| **28** | 0.47 | 1.87 | ND | 4.55 | ND |
| **29** | 0.15 | 1.37 | ND | 15.86 | ND |
| **30** | 0.22 | 2.39 | ND | 1.40 | ND |
| **31** | 0.20 | 1.02 | ND | 1.64 | ND |
| **32** | 2.12 | 1.03 | ND | 2.18 | ND |
| **33** | 1.52 | 1.43 | ND | 5.47 | ND |
| **34** | 0.28 | 1.15 | ND | 7.18 | ND |
| **35** | 5.66 | 1.64 | ND | 1.20 | ND |
| **36** | 0.13 | 1.06 | ND | 1.10 | ND |
| **37** | 1.08 | 1.89 | ND | 1.71 | ND |
| **38** | 0.19 | 1.12 | ND | 1.06 | ND |
| **GSK21264 58** | 0.014 | 0.154 | 0.537 | ND | ND |
| **DOX** | 0.324 | 0.0308 | 0.536 | ND | ND |

As can be seen from Table 5, the compounds of the present invention have good anti-tumor activity on lung cancer cell A549, colon cancer cells HCT116, hepatoma cells HepG2, breast cancer cells ZR-75-30, brain star glioblastoma U87MG. It should be particularly noticed that some compounds such as 1, 9, 10 and 17 havs show high in vitro proliferation inhibition, which laid the foundation of the development of high efficiency, low toxicity and high specific antitumor agents, with good development value.

### Example 41: Cytotoxicity test of Compound 9, 10 and 17 on 60 kinds of human cancer cell lines from the US National Cancer Institute

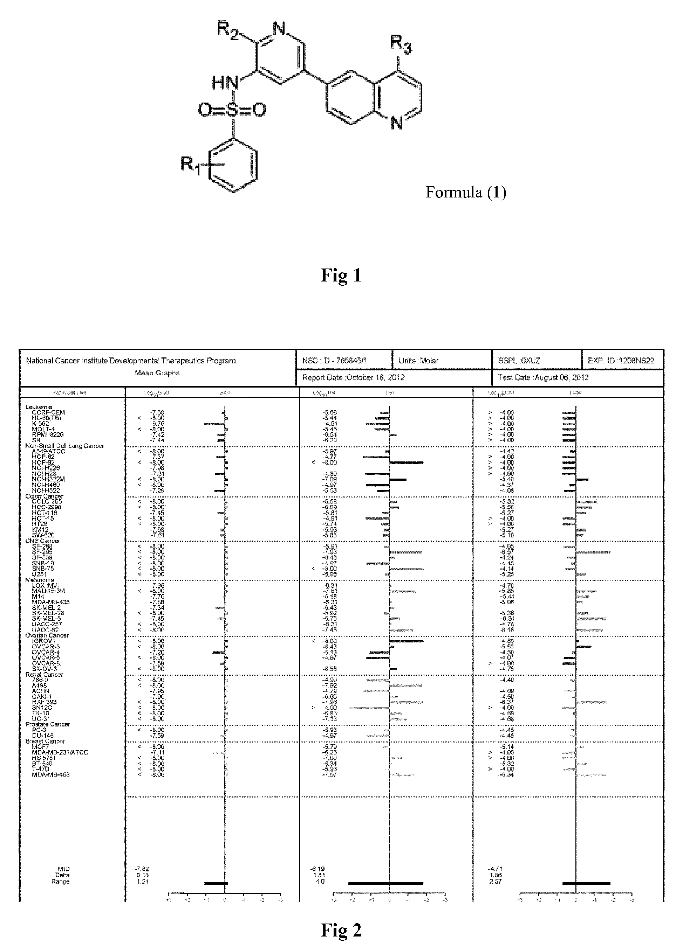

Figure 2 shows the results of the activity of the compound 10. Compound 10 showed good broad-spectrum anti-tumor activity, with an average half growth inhibitory concentration (GI₅₀) less than 10 nM on the 36 tumor cell lines, and an average GI₅₀ of 15.14 nM on the 60 kinds of tumor cells.

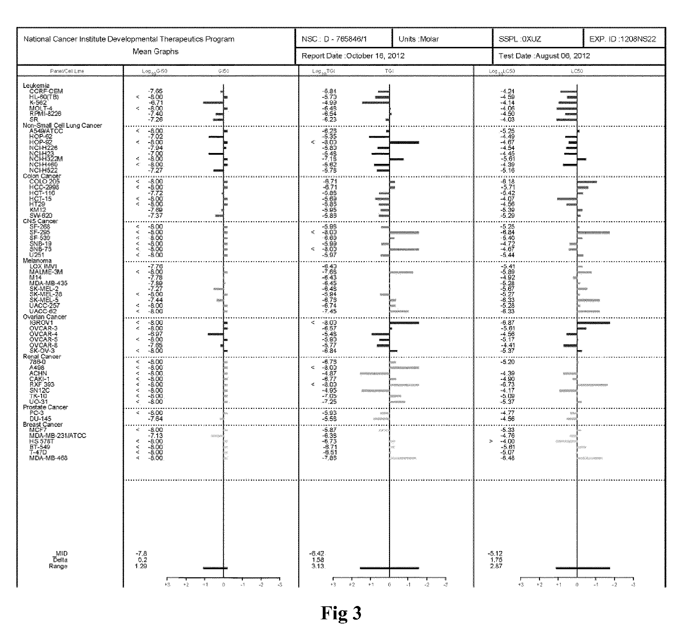

Figure 3 shows the results of the activity of the compound 17. Compound 17 showed good broad-spectrum anti-tumor activity, with an average half growth inhibitory concentration (GI₅₀) less than 10 nM on the 38 tumor cell lines, and an average GI₅₀ of 15.85 nM on the 60 kinds of tumor cells.

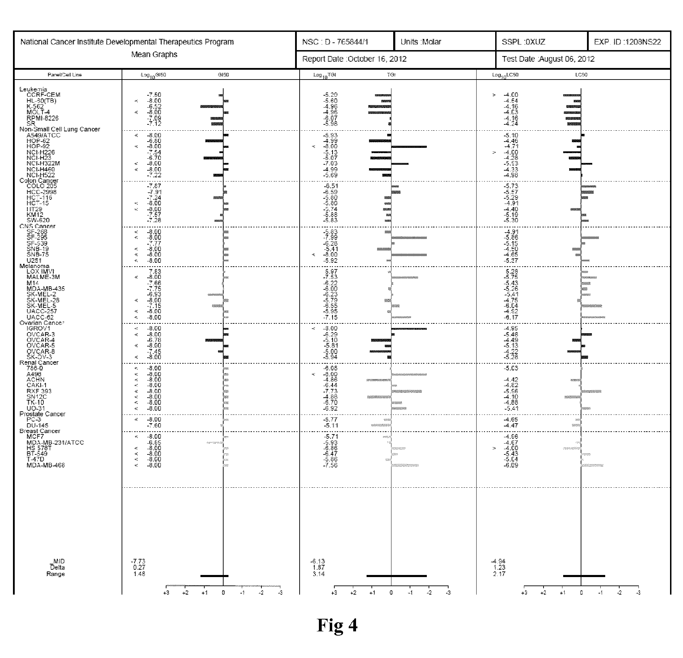

Figure 4 shows the results of the activity of the compound 9. Compound 9 showed good broad-spectrum anti-tumor activity, with an average half growth inhibitory concentration (GI₅₀) less than 10 nM on the 35 tumor cell lines, and an average GI₅₀ of 18.62 nM on the 60 kinds of tumor cells.

**Table 6 Research results of in vitro growth inhibitory activity of compound 9, 10 and 17 on seven kinds of tumor cells (GI₅₀ value: nM)**

| Compound number | A549 | COLO205 | SF539 | OVCAR3 | SN12C | PC3 | MCF7 | Average GI₅₀ |
|---|---|---|---|---|---|---|---|---|
| **9** | <10 | 13.49 | 16.98 | <10 | <10 | <10 | <10 | 18.62 |
| **10** | <10 | <10 | <10 | <10 | <10 | <10 | <10 | 15.14 |
| **17** | <10 | <10 | <10 | <10 | <10 | <10 | <10 | 15.85 |

As can be seen from Table 6, the three compounds selected showed outstanding growth inhibitory activity to a variety of common human tumor cells, the average GI₅₀ to 60 kinds of human tumor cell activity is about 15-19 nM, which is more than twice as the cytotoxic activity of paclitaxel or camptothecin, thus having good development prospects as targeted anticancer drugs, they have.

### Example 42 Studies on in vivo efficacy of compound 9 on human lung carcinoma A549 xenografts in nude mice

### (1) Experimental method

1. Sample preparation:
   Compound 9 and the positive control drug GSK2126458 were hydrotroped with Tween-80, and diluted with distilled water to the desired concentration.
2. Tested animals
   Strain: BALB / C nude mice (SPF grade), Source: Shanghai Slac laboratory animal limited liability company, the production license number: SCXK (Shanghai) 2007-0005, the using license number: SYXK (Shanghai) 2009-0068, gender: male, weight: 18-20g, the number of animals per group: 6.
3. Experimental method
   A549 solid tumors which were well-grown were taken, cut to uniform bits of about 3mm size under aseptic condition, every bit was inoculated to each mouse subcutaneously in the right armpit with trocar, and randomly divided into three groups, each treatment was:
   1) Control (saline), gavage, the dose 25ml / kg, 14 days;
   2) Compound **GSK2126458,** gavage, the dose 3mg / kg, 14 days;
   3) Compound **9,** gavage, the dose 10mg / kg, 14 days;
      Regrouped according to tumor size on the 13th day after inoculation, the animals of which tumor size is too large and too small were eliminated, the average tumor volume of each group are basically the same. Adminstration was carried out according to the above regimen; the dosing volume was 0.5ml / 20g body weight. The axis (a), shorter diameter (b) of tumor was measured 2 times a week with an electronic digital caliper, calculate formula of tumor volume (tumor volume, TV) is as follows: TV = 1 / 2 x a x b2, calculate formula of relative tumor volume (relative tumor volume, RTV), is as follows: RTV = Vt / Vo, wherein Vo is the tumor volume measured at the time when dividing cages (i.e. d1), Vt is the tumor volume measured each time. Evaluation index of anti-tumor activity is relative tumor proliferation rate T / C (%), T / C (%) = TRTV / CRTV x 100%, or relative tumor growth inhibition rate (%) :( 1-T / C) x 100%. T test was conducted. Animals were sacrificed 30 days after inoculation, dissected and the tumor mass were taken, tumor weight was measured.

### (2) Test result

Table 7 in vivo efficacy of compound 9 on human lung carcinoma A549 xenografts in nude mice

| group | dosage | Dosing | Number of Animal wight(g) animals | | | RTV(d18) | Tumor Inhibition rate |
|---|---|---|---|---|---|---|---|
| | (mg/kg) | regimen | start | end | (tumor removed) | *x̅* ±SD | % |
| Control | 25ml/kg | ig×14 | 10 | 10 | 24.96±1.67 | 20.29±3.94 | |
| **GSK2126458** | 3 | ig×11 | 6 | 6 | 21.41±1.49** | 6.91±1.97** | 65.93 |
| **9** | 10 | ig×14 | 6 | 6 | 23.28±1.34 | 5.37±1.86** | 73.52 |

| | | | | | | | |
|---|---|---|---|---|---|---|---|
| Compared with control: **P<0.01. | | | | | | | |

Compound 9 was gavaged by oral for 14 times, at a dose of 10mg / kg of A549, and the inhibition rate to human lung tumor was 73.52%.

Compound **GSK2126458** was gavaged by oral for 9 days, stopped for 3 days, and then administrated for 2 days, and the inhibition rate to human lung tumor at a dose of 3mg / kg of A549 was 65.93%.

As can be seen from anti-tumor test results, the compound 9 has a better in vivo anti-tumor activity than compound **GSK2126458,** and has less infect to the body weight of the animal at the effective dose.

### Example 43 studies on in vivo pharmacokinetics of compound 9

### (1) Test method

### 1. Test animal

SD rats, male, 4-6 weeks old, weighing 200-220 g, the total number is 12, were purchased from Shanghai Slac laboratory animal limited liability company, animal production license number: SCXK (Shanghai) 2008-0016, the experimental animals using license number: SCXK (Shanghai) 2007-0005, certificate number: 2007000514232.

### 2. Regimen and blood

SD rats were randomly divided into two groups, numbered A and B, while 6 rats in each group, before the experiment starts, the rats were fasted 12 hours overnight, group A were tail vein injected with compound 9 by dose 2mg / kg; group B were intragastric administrated with Compound 9 suspensions, of which the dose was calculated as 20mg / kg;
Group A: tail intravenous injection (i.v.) administrated compound 9. Orbital blood 0.3mL was taken on 0, 5, 10, 15, 30, 45, 60min, 2h, 3h, 4h, 6h, 8h, 12h, and was placed in 1.5mL EP tube coated with heparin, shaked up and down for 3 times, centrifuged at 3500rpm for 5min to get supernatant, and was kept in refrigerator at -20 °C to save backup;
Dose was 2mg / kg (2.6mL / kg), administered in a concentration of 770µg / mL, which was dissolved with mixed aqueous solution of 9.4% ethanol and 24.5% isopropanol;
Group B: intragastric administration (i.g.), dose of 20mg / kg, administered in a concentration of 1.5mg / mL, and 3.75% Tween 80 was used to dissolve compound 9, gavage dose was (13.3 mL / kg);
Orbital blood 0.5mL was taken at 0, 5, 10, 15, 30, 45, 60min, 2h, 3h, 4h, 6h, 8h, 12h, 24h, and was placed in 1.5mL EP tube coated with heparin, shaked up and down for 3 times, centrifuged at 3500rpm for 5min to get supernatant, and kept in refrigerator at -20°C to save backup.

### 3. Preparation and analysis of plasma sample

Plasma sample 100µL was precisely measured, put in 1.5mL plastic centrifuge tube, 10µL methanol and 190µL methanol solution of compound 17 (internal standard) of 100.8ng • mL⁻¹ were added, vortex mixed for 30s, centrifuged at 12000r • min⁻¹ for 10min, 200µL supernatant was taken and put into the vial, 5µL injected for HPLC-MS analysis.

Analytical conditions: column: SHISEIDO MG-C18 (100 mm ×3.0 mm, 3.0µm), mobile phase: acetonitrile: water (containing 0.1% formic acid = 25: 75 (V / V); flow rate: 0.8 mT·min⁻¹ analysis time 6 min, column temperature: 25°C, the injection volume: 5µL, split ratio 2: 1. The mass was in ESI source positive ion mode, the capillary voltage was 4000V, drying flow rate was 10 L / min, the atomizing air pressure was 40psi, temperature of the drying gas was 350 °C. Selective ion mode (SIM), the ion of compound 9 was [M + H] ions + m / z 561.2 and that of the internal standard compound 17 [M + H] + m / z 591.2, and the debris voltage was 70eV.

The data gained were substituted into the accompanying standard curve, thus obtaining the drug concentration in rat plasma.

### 4. Data processing

The pharmacokinetic parameters were analyzed by using non-compartmental model, the peak concentration Cmax and peak time Tmax were taken Found value, and the pharmacokinetic parameters were processed to obtain the pharmacokinetic parameters result of single dose.

### (2) Experimental method

### 1. The plasma concentration-time data of compound 9 after intravenous injection of compound 9

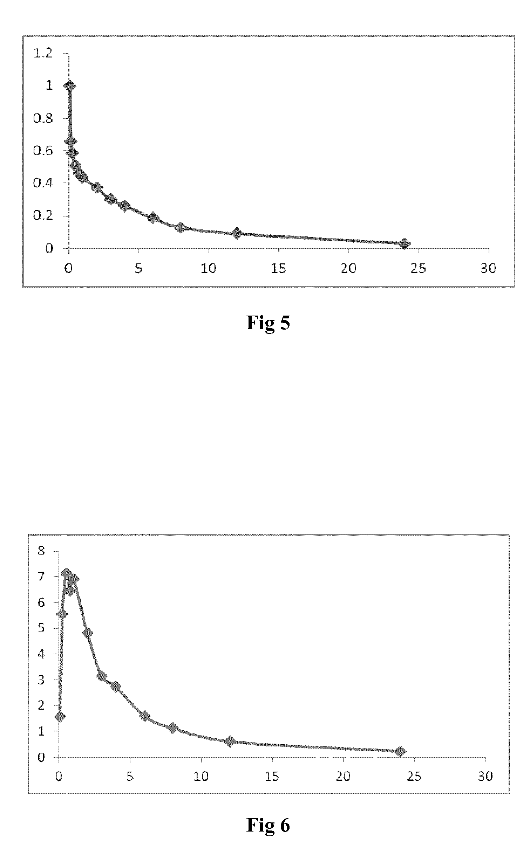

After tail intravenous injection administrated compound 9, concentration - time data in rat plasma of compound 9 is shown in Table 8; average concentration - time curve of compound 9 in rat plasma of the compound 9 tail vein injected group in rat plasma is shown in Fig 5.

**Table 8 pharmacokinetic parameters of concentration of compound 9 in rat plasma after**

| Cmax (µg/mL) | k (h⁻¹) | t_{1/2} (h) | AUC(0-24) (mL/h·h) | AUC (µg/mL·h) | AUMC(0-24) (µg/mL·h) | AUMC (µg/mL·h) | CL (µg/mL) | MRT (h) | Vd (mL) |
|---|---|---|---|---|---|---|---|---|---|
| 0.9992 | 0.1063 | 6.550 | 3.450 | 3.741 | 22.23 | 31.95 | 0.5353 | 8.541 | 5.053 |

### 2. The plasma concentration- time data of compound 9 after oral administration of compound 9

After intragastric administration of compound 9, concentration - time data in rat plasma of compound 9 is shown in Table 9; average concentration - time curve of compound 9 in rat plasma of the compound 9 intragastric administrated group is shown in Fig 6.

**Table 9 pharmacokinetic parameters of concentration of compound 9 in rat plasma after intragastric administration of compound 9**

| Tmax (h) | Cmax (µg/mL) | k (h⁻¹) | t_{1/2} (h) | AUC(0-24) (mL/h·h) | AUC (µg/mL·h) | AUMC(0-24) (µg/mL·h) | AUMC (µg/mL·h) | CL (µg/mL) | MRT (h) | Vd (mL) |
|---|---|---|---|---|---|---|---|---|---|---|
| 0.5 | 7.118 | 0.1573 | 4.406 | 34.07 | 35.63 | 183.0 | 230.2 | 0.5614 | 6.461 | 3.569 |

### 3. Absolute bioavailability of compound 9 when intragastric administered to rats

According to the calculation formula of absolute bioavailability
F0-t= AUC0-t T·Div/AUC0-t iv·DT=34.07×2/3.450/20=98.75%
F0-∞= AUC0-∞T·Div/AUC0-∞iv·DT=35.63×2/3.741/20=95.24%

It can be seen from the results of pharmacokinetic studies that, when intragastric administered, the half-life of Compound 9 is 4.4 hours, the bioavailability is up to 95.24%, of which the pharmacokinetic properties are excellent.

### Example 44 the solubility test of compound 9 in buffer solution at pH = 1

### 1. Test of solubility of compound 9

Compound 9 1.02mg was precisely weighed and placed in 1.5mL of EP tube, and 10uL pH = 1 buffer solution was added, vortexed for 30S and ultrasonic for 20min. There was a lot of visible turbidity after that. 20uL buffer was added and ultrasounded and the solution was found clear and transparent, and compound 9 had been completely dissolved (of which the solubility was 33.3mg / mL).

### 2. Test of solubility of compound GSK2126458

Compound GSK2126458 1.05mg was precisely weighed and placed in 1.5mL of EP tube, and 10uL pH = 1 buffer solution was added, vortexed for 30S and ultrasonic for 20min. There was a lot of visible turbidity after that. 20uL buffer was added and ultrasounded and the solution was found still turbid. Turbidity reduced after the solution was diluted to 1mL and vortexed, while there still exist residual after diluted to 2mL. The turbidity disappeared after diluted to 5ml, and the compound **GSK2126458** was completely dissolved. (the solubility is 0.2mg / mL)

In summary, Compound 9 was dissolved in buffer solution of which pH = 1, the solubility was 33.3mg / mL; Compound **GSK2126458** was very slightly soluble in buffer solution of which pH = 1, the solubility was 0.2mg / mL. In buffer solution of which pH = 1, the solubility of the compound 9 is much larger than that of **GSK2126458.**

### Example 45 Capsule Composition

Dosage forms for oral administration of the present invention were prepared by using the ingredients shown in Table 10 to fill into empty capsules by the proportions shown.

**Table 10 content of each component of the capsules**

| ingredient | Compound in example 1 | lactose | Talc | magnesium stearate |
|---|---|---|---|---|
| content | 25 mg | 55 mg | 16 mg | 4 mg |

### Example 46 Injectable parenteral compositions

Injection for administration of the present invention is made by stirring 1.5% compound of Example I (by weight percent) in a 10% (volume percent) aqueous solution of propylene glycol.

### Example 47 Tablet Composition

The sucrose, calcium sulfate dihydrate and PI3K inhibitor shown in Table 11 were mixed by the ratios shown, and granulated with 10% gelatin solution. The wet granules were screened, dried, mixed with starch, talc and stearic acid, screened and compressed into tablets.

**Table 11 content of each component in the tablet Composition**

| ingredient | Compound in example 1 | calcium sulfate dihydrate | lactose | starch | Talc | stearic acid |
|---|---|---|---|---|---|---|
| content | 20 mg | 30 mg | 4 mg | 2 mg | 1 mg | 0.5 mg |

Above are only preferred embodiments of the present invention, it should be noted that for the ordinary skilled in the art, without departing from the premise, can also make a number of improvements and additions, such modifications and supplements also should be considered as the scope of the present invention.

## Claims

1. A compound represented by formula (1), or the solvate, pharmaceutically acceptable salt, pro-drug or polymorph thereof: 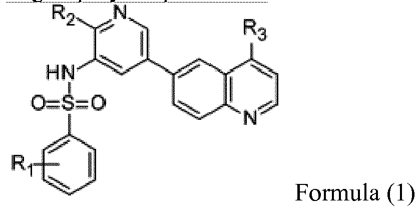 wherein,
R₁ group may be independently selected from the group consisting of: hydrogen, halogen, nitrile group, nitro, hydroxyl, carboxyl, substituted or unsubstituted C1-8 acyl, substituted or unsubstituted amino, substituted or unsubstituted C1-8 alkyl, substituted or unsubstituted C1-8 alkoxy, which may be single, double or multi-substituted;
R₂ group may be independently selected from the group consisting of: hydrogen, substituted or unsubstituted C1-8 alkoxy, or halogen;
R₃ group is selected from any of the following groups: 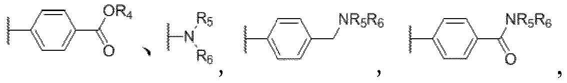 wherein, R₄, R₅, R₆ may be independently selected from the group consisting of: hydrogen, substituted or unsubstituted C1-8 straight or branched alkyl, or R₅ and R₆ may form a ring, with a proviso that R₅ and R₆ are not simultaneously hydrogen; or R₄, R₅ and R₆ being -(CH₂)ₙNR₇R₈, wherein n is 1-8, R₇ and R₈ are independently selected from the group consisting of: hydrogen, substituted or unsubstituted C1-8 straight or branched alkyl, or R₇ and R₈ form a ring;
wherein when R₅ and R₆ form a ring, or R₇ and R₈ form a ring, they may form a nitrogen-containing saturated heterocyclic ring or an aromatic heterocyclic ring with the nitrogen attached, while the heterocyclic ring may be substituted;
the term "substituted" means a group substitution by one or more of the following substituents: C1-5 alkyl, C2-5 alkenyl, C2-5 alkynyl, C1-5 alkoxy, halogen, nitro, cyano, hydroxyl, amino, carboxyl, or oxo.

2. The compound of claim 1, wherein R₁ group is mono-, di- or poly-substituted by halogen.

3. The compound of claim 2, wherein R₁ group is 2, 4-difluoro, 4-fluoro or 2-fluoro-substituted.

4. The compound of claim 1, wherein R₂ group is methoxy or halogen.

5. The compound of claim 1, wherein when R₅ and R₆ form a ring, or R₇ and R₈ form a ring, they form a nitrogen-containing saturated heterocyclic ring or an aromatic heterocyclic ring with the nitrogen attached, which comprises: pyrrolidine, piperidine, piperazine, morpholine, imidazole, 2-methylimidazole, pyrazole, 1*H-*1, 2, 4- triazole.

6. The compound of claim 1, it is selected from the group consisting of:
(1). N-(5-(4-(1*H*-1, 2, 4-triazole-1-yl)quinolin-6-yl)-2-methoxypyridine-3-yl) -2, 4-difluorobenzenesulfonamide;
(2). N-(2-methoxy-5-(4-(pyridin-1-yl)quinolin-6-yl)pyridine -3-yl)-2, 4-difluorobenzenesulfonamide;
(3). N-(2-methoxy-5-(4-morpholine quinolin-6-yl)pyridin-3-yl)- 2, 4-difluorobenzenesulfonamide;
(4). N-(5-(4-chlorinequinolin-6-yl)-2-methoxypyridine-3-yl)-2, 4-difluorobenzenesulfonamide;
(5). N-(5-(4-(4-((diethylamine)methyl)phenyl)quinolin-6-yl)-2-methoxypyridine-3-yl)-2, 4-difluorobenzenesulfonamide;
(6). N-(2-methoxy-5-(4-(p-methylphenyl)quinolin-6-yl)pyridin-3-yl)-2, 4-difluorobenzenesulfonamide;
(7). 4-(6-(5-(2, 4-difluorobenzenesulfonamideyl)-6-methoxypyridine-3-yl)quinolin-4-yl)methyl benzoate;
(8). 4-(6-(5-(2, 4-difluorobenzenesulfonamideyl)-6-methoxypyridine-3-yl)quinolin-4-yl) ethyl benzoate;
(9). N-(5-(4-(4-((dimethylamine)methyl)phenyl)quinolin-6-yl)-2-methoxypyridine-3-yl)-2, 4-difluorobenzenesulfonamide;
(10). N-(5-(4-(4-((1*H*-1, 2, 4-triazole-1-yl)methyl)phenyl)quinolin-6-yl)-2-methoxypyridine-3-yl)- 2, 4-difluorobenzenesulfonamide;
(11). N-(2-methoxy-5-(4-(4-(pyrrolidinyl-1-carbonyl)phenyl) quinolin-6-yl)pyridin-3-yl)- 2, 4-difluorobenzenesulfonamide;
(12). N-(5-(4-(4-((tetrahydropyridinyl-1-yl)methyl)phenyl)quinolin-6-yl)- 2-methoxypyridine -3- yl)-2, 4- difluorobenzenesulfonamide;
(13). N-(5-(4-(4-((morpholino-1-yl)methyl)phenyl)quinolin-6-yl)-2-methoxypyridine-3- yl)- 2, 4- difluorobenzenesulfonamide;
(14). N-(5-(4-(4-((piperidine-1-yl)methyl)phenyl)quinolin-6-yl)-2-methoxypyridine-3-yl)- 2, 4-difluorobenzenesulfonamide;
(15). N-(5-(4-(4-((1*H*-imidazole-1-yl)methyl)phenyl)quinolin-6-yl)-2-methoxypyridine-3-yl)-2, 4-difluorobenzenesulfonamide;
(16). N-(5-(4-(4-((2-methyl-1*H*-imidazole-1-yl)methyl)phenyl)quinolin-6-yl)-2-methoxypyridine-3-yl)-2, 4-difluorobenzenesulfonamide;
(17). N-(5-(4-(4-(((2-hydroxyethyl)(methyl)amino)methyl)phenyl)quinolin-6-yl)-2-methoxypyridine-3-yl)-2, 4-difluorobenzenesulfonamide;
(18). N-(5-(4-(4-((4-isopropylpiperazine-1-yl) methyl) phenyl) quinolin-6-yl)- 2-methoxypyridine-3-yl)-2, 4-difluorobenzenesulfonamide;
(19). N-(5-(4-(4-((4-(2-hydroxyethyl) piperazine-1-yl)methyl)phenyl)quinolin -6-yl)-2-methoxypyridine-3-yl)-2, 4-difluorobenzenesulfonamide;
(20). N-(5-(4-(4-((di(2-hydroxyethyl)amino) methyl)phenyl)quinolin-6-yl) -2-methoxypyridine-3-yl)-2, 4-difluorobenzenesulfonamide;
(21). N-(5-(4-(4-((1*H*-pyrazole-1-yl)methyl)phenyl)quinolin-6-yl)-2-methoxypyridine- 3-yl)-2, 4-difluorobenzenesulfonamide;
(22). N-(5-(4-(4-((cyclohexyl(ethyl)amino)methyl)phenyl)quinolin-6-yl)-2-methoxypyridine-3-yl)-2, 4- difluorobenzenesulfonamide;
(23). N-(5-(4-(4-((4-methylpiperazine-1-yl)methyl)phenyl)quinolin-6-yl)- 2-methoxypyridine -3-yl)- 2, 4- difluorobenzenesulfonamide;
(24). N-(5-(4-(4-(((2-(dimethylamino)ethyl)(methyl)amino))methyl)phenyl)quinolin-6-yl)-2-methoxypyridine-3-yl)-2, 4-difluorobenzenesulfonamide;
(25). N-(5-(4-(1*H* -1, 2, 4-triazole-1-yl)quinolin-6-yl)-2- methoxypyridine-3-yl)- 4-fluorobenzenesulfonamide;
(26). N-(5-(4-(1*H* -1, 2, 4- triazole-1-yl) quinolin-6-yl)- 2-chlorinepyridin- 3-yl)- 2, 4-difluorobenzenesulfonamide;
(27). N-(5-(4-(1*H* -1, 2, 4-triazole-1-yl)quinolin-6-yl)-2- chlorinepyridin-3-yl) -4-fluorobenzenesulfonamide;
(28). N-(5-(4-(4-((morpholino-1-yl)methyl)phenyl)quinolin-6-yl)-2-chlorinepyridin-3-yl)- 4-fluorobenzenesulfonamide;
(29). N-(5-(4-(4-((morpholino-1-yl)methyl)phenyl)quinolin-6-yl)-2-methoxypyridine-3-yl) -4-fluorobenzenesulfonamide;
(30). N-(5-(4-(4-((morpholino-1-yl)methyl)phenyl)quinolin-6-yl)-2-chlorinepyridin-3-yl)- 2, 4-difluorobenzenesulfonamide;
(31). N-(5-(4-(4-((dimethylamine)methyl)phenyl)quinolin-6-yl)-2-methoxypyridine-3-yl)- 4-fluorobenzenesulfonamide;
(32). N-(5-(4-(4-((dimethylamine)methyl)phenyl)quinolin-6-yl)-2-chlorinepyridin-3-yl) -2, 4-difluorobenzenesulfonamide;
(33). N-(5-(4-(4-((1*H*-1, 2, 4-triazole-1-yl)methyl)phenyl)quinolin-6-yl)-2-chlorinepyridin-3-yl) -4-fluorobenzenesulfonamide;
(34). N-(5-(4-(4-((1*H*-1, 2, 4-triazole-1-yl)methyl)phenyl)quinolin-6-yl)-2-methoxypyridine -3- yl) -4-fluorobenzenesulfonamide;
(35). N-(5-(4-(4-((1*H*-1, 2, 4-triazole-1-yl)methyl)phenyl)quinolin-6-yl)-2-chlorinepyridin-3-yl) -2, 4-difluorobenzenesulfonamide;
(36). N-(5-(4-(4-(((2-hydroxyethyl)(methyl)amino)methyl)phenyl)quinolin-6-yl)-2-chlorinepyridin-3-yl)-4-fluorobenzenesulfonamide;
(37). N-(5-(4-(4-(((2-hydroxyethyl)(methyl)amino)methyl)phenyl)quinolin-6-yl)- 2-chlorinepyridin-3-yl)-2, 4-difluorobenzenesulfonamide; and
(38). N-(5-(4-(4-(((2-hydroxyethyl)(methyl)amino)methyl)phenyl)quinolin-6-yl)-2-methoxypyridine-3-yl)-4-fluorobenzenesulfonamide.

7. A preparation method for a compound of formula (IV), which comprises the following steps of: 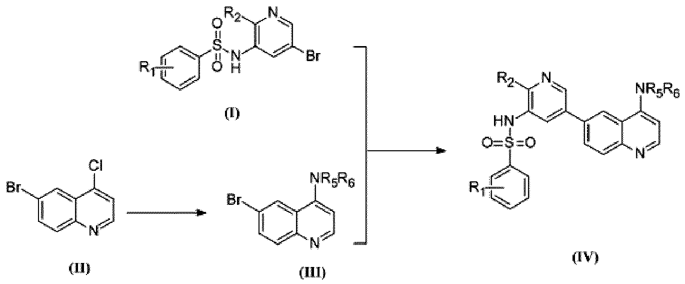
(1) Preparation of intermediate (III)
dissolving 6-bromo-4-chloro-quinoline (II) in DMF, adding various substituted amines, heating and reacting to get intermediate (III);
(2) Preparation of desired product (IV)
intermediate (III) is dissolved in dioxane, and bis (pinacolato) diboron , potassium acetate and palladium catalyst are added;
heating and refluxing under inert gas protected condition until raw materials point of TLC testing disappeares; then the reaction is stopped; adding various substituted brominated aromatic compounds (I) and the other part of palladium catalyst and K₂CO₃ solution, heating and refluxing under inert gas protected condition, thereby forming the desired product **(IV).**

8. A preparation method for the compound of formula (VIII), which comprises the following steps of: 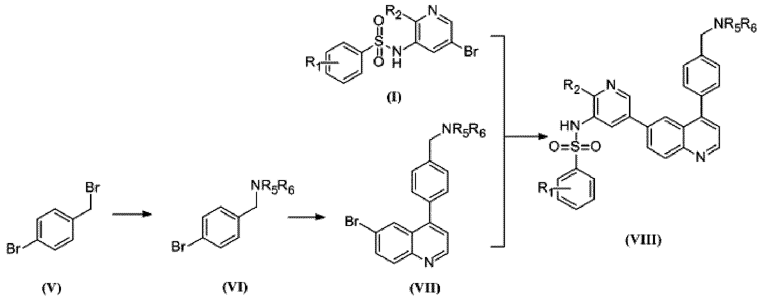
(1) Preparation of compound **(VI)**
dissolving bromine bromide (V) in DMF, adding various nitrogen substituted compounds, stirring overnight under nitrogen protected condition at room temperature to generate compound **(VI);**
(2) Preparation of intermediate **(VII)**
nitrogen-substituted benzyl derivatives **(VI)** are dissolved in dioxane; and bis(pinacolato) diboron, potassium acetate and a palladium catalyst are added; heating and refluxing for several hours under argon protected condition until raw materials point of TLC testing disappears, then stop the reaction; 6-bromo-4-iodo-quinoline, the other part of palladium catalyst and K₂CO₃ solution are added into the mixed system, the reaction is stopped after heating and refluxing for several hours under argon protected condition, then stop the reaction, thereby forming the desired product **(VII);**
(3) Preparation of desired product **(VIII)**
intermediate **(VII)** is dissolved in dioxane, and bis (pinacolato) diboron, potassium acetate and a palladium catalyst are added; heating and refluxing for several hours under argon protected condition until raw materials point of TLC testing disappears, then the reaction is stopped; adding brominate substituted aromatic compound (I), palladium catalyst and K₂CO₃ solution into the mixed system; heating and refluxing for several hours under argon protected condition, thereby forming the desired product **(VIII).**

9. A preparation method for the compound of formula (XII), which comprises the following steps of: 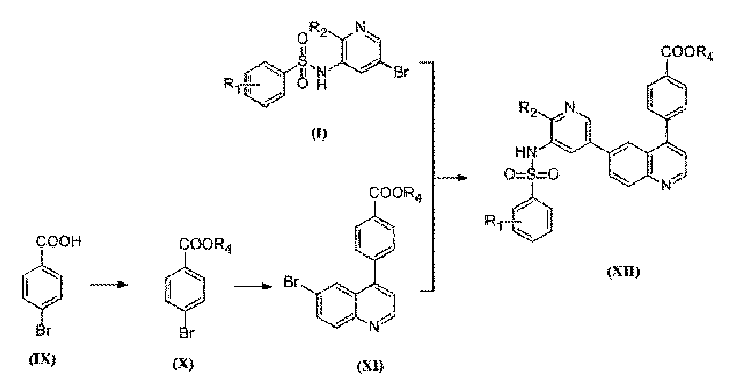
(1) Preparation of p-bromobenzoate ester derivative **(X)**
para-bromobenzoic acid **(IX)** is dissolved in alcohols, and a few droplets of concentrated sulfuric acid is added dropwise, followed by stirring under heating to reflux to form compound (X);
(2) Preparation of intermediate **(XI)**
para-bromobenzoic acid ester derivatives **(X)** are dissolved in dioxane, and bis(pinacolato) diboron, potassium acetate and a palladium catalyst are added; heating and refluxing for several hours under argon protected condition until raw material point of TLC testing disappears, then the reaction is stopped; adding 6-bromo-4-iodo-quinoline, the other part of palladium catalyst and K₂CO₃ solution into the mixed system, heating and refluxing several hours under argon protected condition, and stopping the reaction to obtain the intermediate **(XI);**
(3) Preparation of desired product **(XII)**
intermediate **(XI)** is dissolved in dioxane, and bis (pinacolato) diboron, potassium acetate and a palladium catalyst are added; heating and refluxing for several hours under argon protected condition until raw material point of TLC testing disappears, then the reaction is stopped; adding substituted brominated aromatic compounds **(I),** palladium catalyst and K₂CO₃ solution into the mixed system, heating and refluxing for several hours under argon protected condition, and stopping the reaction to obtain the intermediate **(XII).**

10. A preparation method for the compound of formula (XVI), which comprises the following steps of: 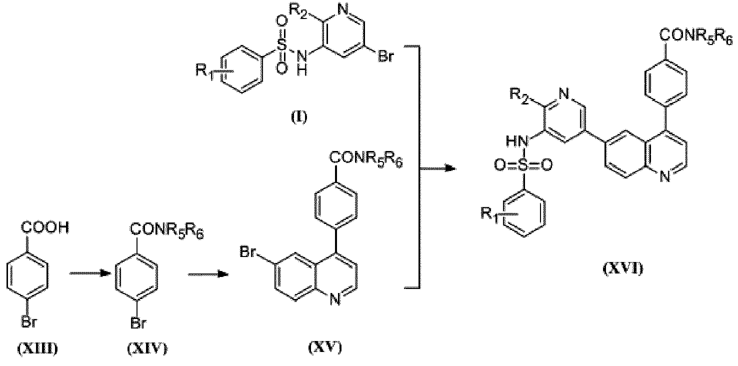
(1) Preparation of para-bromobenzamide derivative **(XIV)**
para-bromobenzoic acid **(XIII)** is dissolved in dichloromethane, EDC/HCl and DMAP are then added; after reacting for two hours, adding another raw material various amine, heating overnight at the room temperature to form compound **(XIV);**
(2) Preparation of the intermediate **(XV)**
para-bromobenzamide derivatives **(XIV)** are dissolved in dioxane, and bis (pinacolato) diboron, potassium acetate and palladium catalyst are added; heating and refluxing for several hours under argon protected condition until raw materials point of TLC testing disappears, then the reaction is stopped; adding 6-bromo-4-iodo-quinoline and another part of palladium catalyst and K₂CO₃ solution into the mixed system, heating and refluxing for several hours under argon protected condition, and stopping the reaction to obtain the intermediate **(XV);**
(3) Preparation of desired product **(XVI)**
intermediate **(XV)** is dissolved in dioxane, and bis (pinacolato) diboron, potassium acetate and a palladium catalyst are added; heating and refluxing for several hours under argon protected condition until raw material point of TLC testing disappears, then the reaction is stopped; adding brominated aromatic compound **(I),** palladium catalyst and K₂CO₃ solution into the mixed system, heating and refluxing for several hours under argon protected condition, and stopping the reaction to obtain the intermediate **(XII).**

11. A pharmaceutical composition comprising the compound of any of claims 1-6 and a pharmaceutically acceptable carrier.

12. A pharmaceutical composition comprising the compound of any of claims 1-6 and an anti-tumor compound selected from the group consisting of: anti-microtubule agents, platinum coordination complexes, alkylating agents, antibiotics, topoisomerase II inhibitors, antimetabolites, topoisomerase I inhibitors, hormones or hormonal analogues, signal transduction pathway inhibitors, non-receptor tyrosine kinase angiogenesis inhibitors, immunosuppressive agents, pro-apoptotic inhibitors and cell cycle signal transduction inhibitors.

13. A use of the compound of any of claims 1-6 for preparation of phosphatidylinositol 3-kinase inhibiting drug.

14. The use of claim 13, wherein the drug is anti-tumor drug or immunosuppressive drug.

15. The use of claim 14, wherein the tumor is lung cancer, leukemia, colon cancer, kidney cancer, prostate cancer, melanoma, liver cancer, ovarian cancer, breast cancer or brain cancer.
